# EUROPEAN PATENT APPLICATION

(11) **EP 4 033 249 A2**
(43) Date of publication of application: **27.07.2022**
(21) Application number: 20865273.5
(22) Date of filing: 18.09.2020
(51) Int. Cl.: G01N 33/68, A61K 48/00, A61K 31/7088, A61P 31/10, C07K 16/14, C12Q 1/18, A61K 39/00

(54) **USE OF GENE INVOLVED IN PASSAGE THROUGH BRAIN-BLOOD BARRIER AND SURVIVAL INSIDE BRAIN OF CAUSATIVE FUNGI OF MENINGOENCEPHALITIS**

(30) Priority: 18.09.2019 KR 20190114797
(71) Applicant: Amtixbio Co., Ltd., Seoul 05836 (KR)
(72) Inventor: BAHN, Yong-Sun, Seoul 05538 (KR); CHEONG, Eunji, Seoul 07983 (KR); LEE, Jong Seung, Seoul 01738 (KR); LEE, Kyung-Tae, Seoul 03651 (KR); LEE, Dong-Gi, Seoul 04075 (KR); HONG, Joohyeon, Seoul 03726 (KR)
(74) Representative: Bringer IP
(86) International application number: PCT/KR2020/012663
(87) International publication number: WO 2021/054786

(57) **Abstract**

The present invention relates to: a method for screening an antifungal agent, the method measuring the amount or activity of proteins involved in passage through the brain-blood barrier; a biomarker composition for diagnosing meningoencephalitis or cryptococcosis; a diagnostic kit including said composition; and a therapeutic pharmaceutical composition including an inhibitor for the protein.

<110>     AMTIXBIO CO., LTD.

<120>     USE OF THE GENES ASSOCIATED WITH BLOOD-BRAIN BARRIER PASSAGE AND
          SURVIVAL IN BRAIN TISSUES OF MENINGOENCEPHALITIS-CAUSING FUNGI

<130>     AMTIX20P02WO

<150>     KR 10-2019-0114797
<151>     2019-09-18

<160>     78

<170>     KoPatentIn 3.0

<210>     1
<211>     20
<212>     DNA
<213>     Artificial Sequence

<220>
<223>     qPCR primer 1 of URE1


<400>     1
ttgctttccc tttcttcctc                                                   20


<210>     2
<211>     19
<212>     DNA
<213>     Artificial Sequence

<220>
<223>     qPCR primer 2 of URE1


<400>     2
ggtggtgagg atgagtttg                                                    19


<210>     3
<211>     19
<212>     DNA
<213>     Artificial Sequence

<220>
<223>     qPCR primer 1 of CPS1


<400>     3
actcacggat gactgtttg                                                    19


<210>     4
<211>     19
<212>     DNA
<213>     Artificial Sequence

<220>
<223>     qPCR primer 2 of CPS1


<400>     4

atacaccgt caatgtgtg                                                    19


<210>    5
<211>    20
<212>    DNA
<213>    Artificial Sequence

<220>
<223>    qPCR primer 1 of ITR1a


<400>    5
cttcaaccga ggtcatactc                                                  20


<210>    6
<211>    19
<212>    DNA
<213>    Artificial Sequence

<220>
<223>    qPCR primer 2 of ITR1a


<400>    6
agattccgat accaagggc                                                   19


<210>    7
<211>    20
<212>    DNA
<213>    Artificial Sequence

<220>
<223>    qPCR primer 1 of ITR3c


<400>    7
ccctttggtc aggtgatttc                                                  20


<210>    8
<211>    21
<212>    DNA
<213>    Artificial Sequence

<220>
<223>    qPCR primer 2 of ITR3c


<400>    8
gctgaaatag ggatggaaca g                                                21


<210>    9
<211>    20
<212>    DNA
<213>    Artificial Sequence

<220>
<223>    qPCR primer 1 of MPR1

<400>    9
cgaggttctt gatgatgctg                                                    20


<210>    10
<211>    20
<212>    DNA
<213>    Artificial Sequence

<220>
<223>    qPCR primer 2 of MPR1


<400>    10
atccgaggaa agtctgagcc                                                    20


<210>    11
<211>    20
<212>    DNA
<213>    Artificial Sequence

<220>
<223>    qPCR primer 1 of PLB1


<400>    11
gttgcccaca aatcttcttg                                                    20


<210>    12
<211>    20
<212>    DNA
<213>    Artificial Sequence

<220>
<223>    qPCR primer 2 of PLB1


<400>    12
gcgttggttt cagtgtaaag                                                    20


<210>    13
<211>    20
<212>    DNA
<213>    Artificial Sequence

<220>
<223>    qPCR primer 1 of FNX1


<400>    13
cctgttggtg gatggattag                                                    20


<210>    14
<211>    21
<212>    DNA
<213>    Artificial Sequence

<220>

<223>    qPCR primer 2 of FNX1

<400>    14
ggaaccacaa caaaggtgta g                                                21

<210>    15
<211>    20
<212>    DNA
<213>    Artificial Sequence

<220>
<223>    qPCR primer 1 of RUB1

<400>    15
aagaacgggt tgaagagaag                                                  20

<210>    16
<211>    20
<212>    DNA
<213>    Artificial Sequence

<220>
<223>    qPCR primer 2 of RUB1

<400>    16
agtcttggat ggttttgtcg                                                  20

<210>    17
<211>    20
<212>    DNA
<213>    Artificial Sequence

<220>
<223>    qPCR primer 1 of ADA2

<400>    17
tgatgccgaa atggctgtaa                                                  20

<210>    18
<211>    19
<212>    DNA
<213>    Artificial Sequence

<220>
<223>    qPCR primer 2 of ADA2

<400>    18
ttcatctgga ggacgagtg                                                   19

<210>    19
<211>    21
<212>    DNA
<213>    Artificial Sequence

<220>
<223>    qPCR primer 1 of BZP2


<400>    19
tgcatgatga tgatcagcag g                                                21


<210>    20
<211>    19
<212>    DNA
<213>    Artificial Sequence

<220>
<223>    qPCR primer 2 of BZP2


<400>    20
gtaatgtcag tgagtgcgg                                                   19


<210>    21
<211>    18
<212>    DNA
<213>    Artificial Sequence

<220>
<223>    qPCR primer 1 of HAP2


<400>    21
cccagcccgg gccatcga                                                    18


<210>    22
<211>    20
<212>    DNA
<213>    Artificial Sequence

<220>
<223>    qPCR primer 2 of HAP2


<400>    22
gcgtcacttt ggacatactc                                                  20


<210>    23
<211>    21
<212>    DNA
<213>    Artificial Sequence

<220>
<223>    qPCR primer 1 of PHO4/HLH3


<400>    23
ccgatgctta aacctacgca t                                                21


<210>    24
<211>    22

<212>    DNA
<213>    Artificial Sequence

<220>
<223>    qPCR primer 2 of PHO4/HLH3


<400>    24
caggatgata cttttggact gc                                                22


<210>    25
<211>    19
<212>    DNA
<213>    Artificial Sequence

<220>
<223>    qPCR primer 1 of FZC6


<400>    25
gtggcggcgg cggagacgg                                                    19


<210>    26
<211>    19
<212>    DNA
<213>    Artificial Sequence

<220>
<223>    qPCR primer 2 of FZC6


<400>    26
gcttggtcgg ataaagatg                                                    19


<210>    27
<211>    19
<212>    DNA
<213>    Artificial Sequence

<220>
<223>    qPCR primer 1 of FAP1


<400>    27
acagacctcc gctcgttgt                                                    19


<210>    28
<211>    19
<212>    DNA
<213>    Artificial Sequence

<220>
<223>    qPCR primer 2 of FAP1


<400>    28
gcttttgcct gctattgac                                                    19

<210>	29
<211>	20
<212>	DNA
<213>	Artificial Sequence

<220>
<223>	qPCR primer 1 of JJJ1


<400>	29
atgatcccaa atcgaagctg	20


<210>	30
<211>	20
<212>	DNA
<213>	Artificial Sequence

<220>
<223>	qPCR primer 2 of JJJ1


<400>	30
gtatcatcca ttttcctggc	20


<210>	31
<211>	20
<212>	DNA
<213>	Artificial Sequence

<220>
<223>	qPCR primer 1 of ERT1


<400>	31
ctgttttccc tggtctcaac	20


<210>	32
<211>	19
<212>	DNA
<213>	Artificial Sequence

<220>
<223>	qPCR primer 2 of ERT1


<400>	32
gacagagttg ttcacgagg	19


<210>	33
<211>	19
<212>	DNA
<213>	Artificial Sequence

<220>
<223>	qPCR primer 1 of FZC1


<400>	33
atcggagttc atcagcagc	19

<210>    34
<211>    19
<212>    DNA
<213>    Artificial Sequence

<220>
<223>    qPCR primer 2 of FZC1


<400>    34
agtgggaatc gtcgttgac                                                    19


<210>    35
<211>    22
<212>    DNA
<213>    Artificial Sequence

<220>
<223>    qPCR primer 1 of FZC9


<400>    35
atcccgctgg caaagagaca tc                                                22


<210>    36
<211>    20
<212>    DNA
<213>    Artificial Sequence

<220>
<223>    qPCR primer 2 of FZC9


<400>    36
ctttttccg ctggtttctc                                                    20


<210>    37
<211>    19
<212>    DNA
<213>    Artificial Sequence

<220>
<223>    qPCR primer 1 of FZC31


<400>    37
aaatgtcccg aaaaggaag                                                    19


<210>    38
<211>    21
<212>    DNA
<213>    Artificial Sequence

<220>
<223>    qPCR primer 2 of FZC31

<400>    38
tctcttcttc ttctgacctg c                                                21

<210>    39
<211>    20
<212>    DNA
<213>    Artificial Sequence

<220>
<223>    qPCR primer 1 of PDR802

<400>    39
tttcgtagcc tgtaagtggc                                                  20

<210>    40
<211>    19
<212>    DNA
<213>    Artificial Sequence

<220>
<223>    qPCR primer 2 of PDR802

<400>    40
ggaacattgg gaaaaggtg                                                   19

<210>    41
<211>    20
<212>    DNA
<213>    Artificial Sequence

<220>
<223>    qPCR primer 1 of HOB1

<400>    41
cctcgcaagt tccccagcta                                                  20

<210>    42
<211>    20
<212>    DNA
<213>    Artificial Sequence

<220>
<223>    qPCR primer 2 of HOB1

<400>    42
tgtatgaggt cttgtccacc                                                  20

<210>    43
<211>    20
<212>    DNA
<213>    Artificial Sequence

<220>
<223>    qPCR primer 1 of SRE1

<400>    43
gaacaagact cgagcttcgc                                                    20


<210>    44
<211>    19
<212>    DNA
<213>    Artificial Sequence

<220>
<223>    qPCR primer 2 of SRE1


<400>    44
cgaaggggaa ctggttatc                                                     19


<210>    45
<211>    20
<212>    DNA
<213>    Artificial Sequence

<220>
<223>    qPCR primer 1 of HXL1


<400>    45
gacgtcaagc caataatcga                                                    20


<210>    46
<211>    19
<212>    DNA
<213>    Artificial Sequence

<220>
<223>    qPCR primer 2 of HXL1


<400>    46
cgttctccgt cttgatagc                                                     19


<210>    47
<211>    20
<212>    DNA
<213>    Artificial Sequence

<220>
<223>    qPCR primer 1 of NRG1


<400>    47
gcaacccccg tacaggtatc                                                    20


<210>    48
<211>    20
<212>    DNA
<213>    Artificial Sequence

<220>
<223>    qPCR primer 2 of NRG1


<400>    48
gctatgttca ggagcgatac                                                    20


<210>    49
<211>    17
<212>    DNA
<213>    Artificial Sequence

<220>
<223>    qPCR primer 1 of GAT201


<400>    49
catcccgtcg ccacagc                                                       17


<210>    50
<211>    19
<212>    DNA
<213>    Artificial Sequence

<220>
<223>    qPCR primer 2 of GAT201


<400>    50
ggagtatggc tgaaatctg                                                     19


<210>    51
<211>    20
<212>    DNA
<213>    Artificial Sequence

<220>
<223>    qPCR primer 1 of ACT1


<400>    51
gttcgagact ttcaatgccc                                                    20


<210>    52
<211>    20
<212>    DNA
<213>    Artificial Sequence

<220>
<223>    qPCR primer 2 of ACT1


<400>    52
accagagtca agaacgatac                                                    20


<210>    53
<211>    19
<212>    DNA

<213>    Artificial Sequence

<220>
<223>    qPCR primer 1 of R265_ITR1


<400>    53
gctggtcatc atcaaggac                                                    19


<210>    54
<211>    19
<212>    DNA
<213>    Artificial Sequence

<220>
<223>    qPCR primer 2 of R265_ITR1


<400>    54
caagacctcc tagtaatgc                                                    19


<210>    55
<211>    20
<212>    DNA
<213>    Artificial Sequence

<220>
<223>    qPCR primer 1 of R265_ITR3


<400>    55
cgtcttccag cgtatttatc                                                   20


<210>    56
<211>    20
<212>    DNA
<213>    Artificial Sequence

<220>
<223>    qPCR primer 2 of R265_ITR3


<400>    56
cagactgaag tgcggttgtg                                                   20


<210>    57
<211>    19
<212>    DNA
<213>    Artificial Sequence

<220>
<223>    qPCR primer 1 of R265_MPR1


<400>    57
cacactctca agcccaaac                                                    19


<210>    58

<211>	19
<212>	DNA
<213>	Artificial Sequence

<220>
<223>	qPCR primer 2 of R265_MPR1


<400>	58
gagcaggaac atcagatac	19


<210>	59
<211>	20
<212>	DNA
<213>	Artificial Sequence

<220>
<223>	qPCR primer 1 of R265_FZC9


<400>	59
cgaccagtta catctatggc	20


<210>	60
<211>	20
<212>	DNA
<213>	Artificial Sequence

<220>
<223>	qPCR primer 2 of R265_FZC9


<400>	60
ggtctgtcaa gctttcgggt	20


<210>	61
<211>	18
<212>	DNA
<213>	Artificial Sequence

<220>
<223>	qPCR primer 1 of R265_FZC31


<400>	61
ttggactacc acaaacgg	18


<210>	62
<211>	18
<212>	DNA
<213>	Artificial Sequence

<220>
<223>	qPCR primer 2 of R265_FZC31


<400>	62
aaatccgcca aaggttca	18

<210>    63
<211>    19
<212>    DNA
<213>    Artificial Sequence

<220>
<223>    qPCR primer 1 of R265_PDR802


<400>    63
ctggcaataa atcctggtc                                                    19


<210>    64
<211>    19
<212>    DNA
<213>    Artificial Sequence

<220>
<223>    qPCR primer 2 of R265_PDR802


<400>    64
tgtgattgct gttcgttga                                                    19


<210>    65
<211>    18
<212>    DNA
<213>    Artificial Sequence

<220>
<223>    qPCR primer 1 of R265_HOB1


<400>    65
attcgcaaca ccatcctc                                                     18


<210>    66
<211>    20
<212>    DNA
<213>    Artificial Sequence

<220>
<223>    qPCR primer 2 of R265_HOB1


<400>    66
gatggaccgg cgacagaagt                                                   20


<210>    67
<211>    18
<212>    DNA
<213>    Artificial Sequence

<220>
<223>    qPCR primer 1 of R265_SRE1


<400>    67

gccaccattt tgataggg                                                    18


<210>    68
<211>    20
<212>    DNA
<213>    Artificial Sequence

<220>
<223>    qPCR primer 2 of R265_SRE1


<400>    68
ctctcctgaa tgtctcaagc                                                  20


<210>    69
<211>    19
<212>    DNA
<213>    Artificial Sequence

<220>
<223>    qPCR primer 1 of R265_HXL1


<400>    69
cccatcagaa atcgttagc                                                   19


<210>    70
<211>    20
<212>    DNA
<213>    Artificial Sequence

<220>
<223>    qPCR primer 2 of R265_HXL1


<400>    70
aggtgaaggc tcgattatgg                                                  20


<210>    71
<211>    19
<212>    DNA
<213>    Artificial Sequence

<220>
<223>    qPCR primer 1 of R265_NRG1


<400>    71
caccaatcca ggtcctaac                                                   19


<210>    72
<211>    21
<212>    DNA
<213>    Artificial Sequence

<220>
<223>    qPCR primer 2 of R265_NRG1

<400>    72
gttgagtcat agccacccca g                                                21

<210>    73
<211>    20
<212>    DNA
<213>    Artificial Sequence

<220>
<223>    qPCR primer 1 of R265_GAT201

<400>    73
cttcatcacc cagatttcag                                                  20

<210>    74
<211>    20
<212>    DNA
<213>    Artificial Sequence

<220>
<223>    qPCR primer 2 of R265_GAT201

<400>    74
caaactcatg ttgttattct                                                  20

<210>    75
<211>    18
<212>    DNA
<213>    Artificial Sequence

<220>
<223>    qPCR primer 1 of R265_PHO4

<400>    75
gccacaagca gattcaag                                                    18

<210>    76
<211>    20
<212>    DNA
<213>    Artificial Sequence

<220>
<223>    qPCR primer 2 of R265_PHO4

<400>    76
agaaatattt gagctttgtc                                                  20

<210>    77
<211>    19
<212>    DNA
<213>    Artificial Sequence

<220>

<223>    qPCR primer 1 of R265_ACT1

<400>    77
cggtatcgtc acaaactgg                                                    19

<210>    78
<211>    19
<212>    DNA
<213>    Artificial Sequence

<220>
<223>    qPCR primer 2 of R265_ACT1

<400>    78
taagaagaac ggggtgctc                                                    19

## Description

### [Technical Field]

The present invention relates to a method for screening an antifungal agent, in which the method includes measuring the amount or activity of proteins involved in passage through the brain-blood barrier (hereinafter referred to as BBB). In addition, the present invention relates to a biomarker composition for diagnosing meningoencephalitis or cryptococcosis, a diagnostic kit including the composition, and a therapeutic pharmaceutical composition including an inhibitor for the protein.

### [Background Art]

*Cryptococcus neoformans* is a fungal pathogen that is distributed in a variety of natural environments, including soil, wood, and algal feces, has a variety of sources of infection, and uses a variety of hosts from lower eukaryotes to aquatic and terrestrial animals. *Cryptococcus neoformans* is the leading cause of death due to fungal meningoencephalitis, which is known to cause approximately one million new infections and approximately 600,000 deaths worldwide each year. However, only limited treatments are being used to treat meningoencephalitis or cryptococcosis.

In the meantime, in order to understand the pathogenic mechanisms of *Cryptococcus neoformans*, extensive research has been conducted over the past decades. In addition to efforts to analyze the function of individual genes/proteins, many additional pathogenic-associated signaling elements have recently been discovered through systematic analysis of large-scale gene deletion mutant libraries. In the pathogenic mechanism of *Cryptococcus neoformans,* passage and proliferation of the BBB is one of the important factors in which *Cryptococcus neoformans* causes lethal damage to mammalian brain tissue.

Nevertheless, factors known to regulate BBB passage and brain infection in *Cryptococcus neoformans* and the complex signaling pathways controlling the same have not yet been elucidated. Accordingly, in relation to the BBB passage of *Cryptococcus neoformans* and regulation of brain infection, it is necessary to fully understand the network of signals and metabolism controlling the pathogenicity of *Cryptococcus neoformans,* and to develop new antifungal targets and drugs.

### [Disclosure]

### [Technical Problem]

An aspect of the present invention is directed to providing a method for screening an antifungal agent.

Another aspect of the present invention is directed to providing a method for screening an antifungal agent for co-administration.

Yet another aspect of the present invention is directed to providing a biomarker composition for diagnosing meningoencephalitis or cryptococcosis.

Yet another aspect of the present invention is directed to providing a kit for diagnosing meningoencephalitis or cryptococcosis.

Yet another aspect of the present invention is directed to providing a pharmaceutical composition for treating meningoencephalitis or cryptococcosis.

### [Technical Solution]

An embodiment of the present invention provides a method for screening an antifungal agent, in which the method includes: (a) contacting a sample to be analyzed with *Cryptococcus neoformans* cells containing an antifungal agent target protein; (b) measuring an amount or activity of the target protein; and (c) discriminating that the sample is an antifungal agent when it is measured that the amount or activity of the antifungal agent target protein is down-regulated in the prior stage, in which the target protein is a protein involved in passage through the BBB. In a related example, the antifungal agent may be an antifungal agent for treating, preventing, or treating and preventing meningoencephalitis or cryptococcosis. In another related embodiment, stages (a) and (b) may be preferably performed at 30°C to 40°C. In another related example, the protein involved in passage through the BBB may be any one or more proteins selected from the group consisting of Cexl, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1. In yet another related example, any one protein selected from the group consisting of Cexl, Alkl, Pbs2, Pkh201, Met3, Hsl101, Snfl, Vrk1, Gal83, Irk2, Hap2, Srel, Fzc1, Pdr802, Fzc9, and Hob1 among the proteins involved in passage through the BBB is a protein involved in the BBB adhesion; and any one protein selected from the group consisting of Alkl, Pkh201, Met3, Hsl101, Snfl, Gal83, Urk1, Irk2, Vrk1, Ada2, Hap2, Srel, Pdr802, and Hob1 among the proteins involved in passage through the BBB is a protein involved in survival inside the brain.

Yet another example of the present invention provides a method for screening an antifungal agent for co-administration, in which the method includes: (a) a first measurement stage of contacting an antifungal agent with *Cryptococcus neoformans* cells containing an antifungal agent target protein, and measuring an amount or activity of the protein; (b) a second measurement stage of contacting a sample to be analyzed and the antifungal agent with *Cryptococcus neoformans* cells containing an antifungal agent target protein, and measuring an amount or activity of the protein; and (c) comparing measured values of the first and second measurement stages, and when the measured value of the second measurement stage is down-regulated from the measured value of the first measurement stage, discriminating that the sample is an antifungal agent for co-administration. In a related example, the antifungal agent in stage (a) may be, for example, one or more antifungal agents selected from the group consisting of fluconazole, itraconazole, voriconazole, and ketoconazole. In another related example, the non-azole-based antifungal agent may be, for example, amphotericin B or fludioxonil. In another related example, the protein involved in passage through the BBB may be any one protein selected from the group consisting of Cexl, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1. In yet another related example, any one protein selected from the group consisting of Cexl, Alkl, Pbs2, Pkh201, Met3, Hsl101, Snfl, Vrk1, Gal83, Irk2, Hap2, Srel, Fzc1, Pdr802, Fzc9, and Hob1 among the proteins involved in passage through the BBB is a protein involved in the BBB adhesion; and any one protein selected from the group consisting of Alkl, Pkh201, Met3, Hsl101, Snfl, Gal83, Urk1, Irk2, Vrk1, Ada2, Hap2, Srel, Pdr802, and Hob1 among the proteins involved in passage through the BBB is a protein involved in survival inside the brain. In another example within the scope of this embodiment, the antifungal agent for co-administration may be for preventing, treating or preventing and treating meningoencephalitis or cryptococcosis.

Yet another example of the present invention provides a biomarker composition for diagnosing meningoencephalitis or cryptococcosis, in which the composition includes any one or more proteins involved in passage through the brain-blood barrier selected from the group consisting of Cexl, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1. One related example of the present invention provides a biomarker composition for diagnosing meningoencephalitis or cryptococcosis, in which when an amount or activity of any one or more proteins among the proteins involved in passage through the brain-blood barrier is down-regulated, it is diagnosed with meningoencephalitis or cryptococcosis. Another related example of the present invention provides a kit for diagnosing meningoencephalitis or cryptococcosis, in which the kit includes a biomarker composition for diagnosing meningoencephalitis or cryptococcosis.

Yet another example of the present invention provides an antifungal pharmaceutical composition including an inhibitor for one or more proteins involved in passage through the BBB selected from the group consisting of Cexl, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1 of *Cryptococcus neoformans.* In a related example, the inhibitor may be any one or more of an antibody, a dominant-negative mutation, and a ribozyme against the protein involved in passage through the BBB. In another related example, the inhibitor may be an antisense oligonucleotide, siRNA, shRNA, miRNA, or a vector containing the same for a gene encoding the protein involved in passage through the BBB. One related example of the present invention includes the pharmaceutical composition and provides a pharmaceutical composition for treating, preventing, or treating and preventing meningoencephalitis or cryptococcosis.

Yet another example of the present invention provides a method for screening a fungal BBB passage inhibitor, in which the method includes: (a) contacting a sample to be analyzed with *Cryptococcus neoformans* cells containing any one or more proteins selected from the group consisting of Cexl, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1; (b) measuring an amount or activity of the protein and/or a gene encoding the protein; and (c) discriminating that the sample is a fungal BBB passage inhibitor when it is measured that the amount or activity of the protein and/or the gene is down-regulated in stage (b). In a related example, stages (a) and (b) may be performed at 30°C to 40°C. Yet another related embodiment of the present invention provides an antifungal composition including an inhibitor screened according to the method, in which the inhibitor may be any one or more of an antibody, a dominant-negative mutation, and a ribozyme against the protein involved in passage through the BBB. In addition, the inhibitor may be an antisense oligonucleotide, siRNA, shRNA, miRNA, or a vector containing the same for a gene encoding the protein involved in passage through the BBB. Yet another related example of the present invention provides an antifungal pharmaceutical composition for treating, preventing, or treating and preventing meningoencephalitis or cryptococcosis, in which the antifungal composition is included as a pharmacologically active ingredient. Yet another related example of the present invention provides a cosmetic composition including the antifungal composition.

Yet another example of the present invention provides a method for screening a bacterial or fungal brain-blood barrier passage inhibitor, in which the method includes: treating a sample to be analyzed with any one or more proteins involved in passage through a brain-blood barrier selected from the group consisting of Cexl, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1 or a gene encoding the protein; and analyzing an amount or activity of any one or more of the proteins or analyzing an amount or activity of any one or more of the genes. Another related example of the present invention provides an antifungal composition including an inhibitor screened according to the method. In this connection, the inhibitor may be any one or more of an antibody, a dominant-negative mutation, and a ribozyme against the protein involved in passage through the BBB. In addition, the inhibitor may be an antisense oligonucleotide, siRNA, shRNA, miRNA, or a vector containing the same for a gene encoding the protein involved in passage through the BBB. Yet another related example of the present invention provides a pharmaceutical composition for preventing, treating, or preventing and treating meningoencephalitis or cryptococcosis, in which the antifungal composition is included as a pharmacologically active ingredient. Yet another related example of the present invention provides a cosmetic composition including the antifungal composition.

### [Advantageous Effects]

The protein of the present invention involved in passage through the brain-blood barrier can be utilized as a new target for alleviation and treatment of meningoencephalitis or cryptococcosis caused by *Cryptococcus neoformans* infection, and can be effectively utilized for screening antifungal agents or drugs capable of inhibiting the protein. It should be understood that the effects of the present invention are not particularly limited to those described above, and the present invention includes all effects that can be deduced from the detailed description of the invention or the configurations of the invention described in the claims.

### [Description of Drawings]

FIG. 1 shows a schematic diagram for comparison of lung and brain-STM analysis.
FIG. 2 shows lung-STM scores using mice inhaled nasally and brain-STM scores using mice injected intravenously.
FIG. 3 shows a schematic diagram for NanoString analysis.

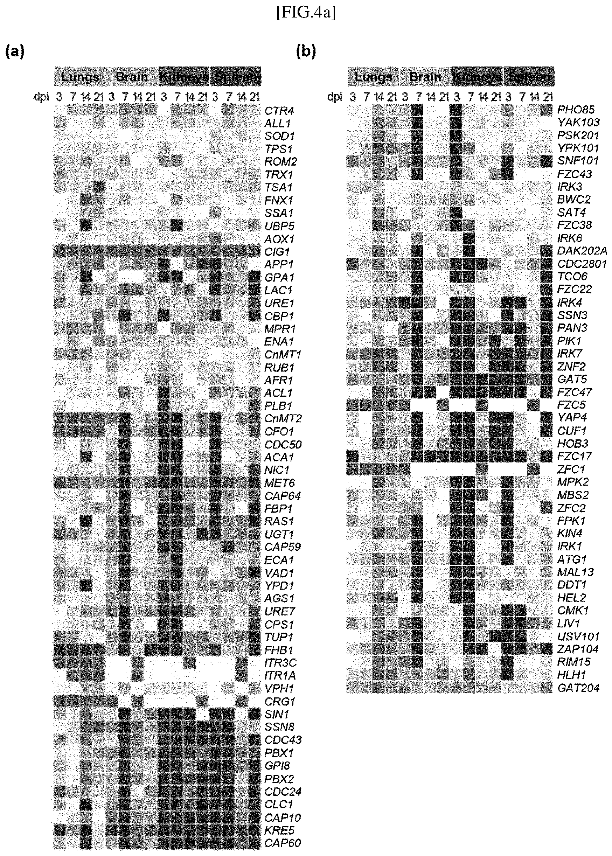

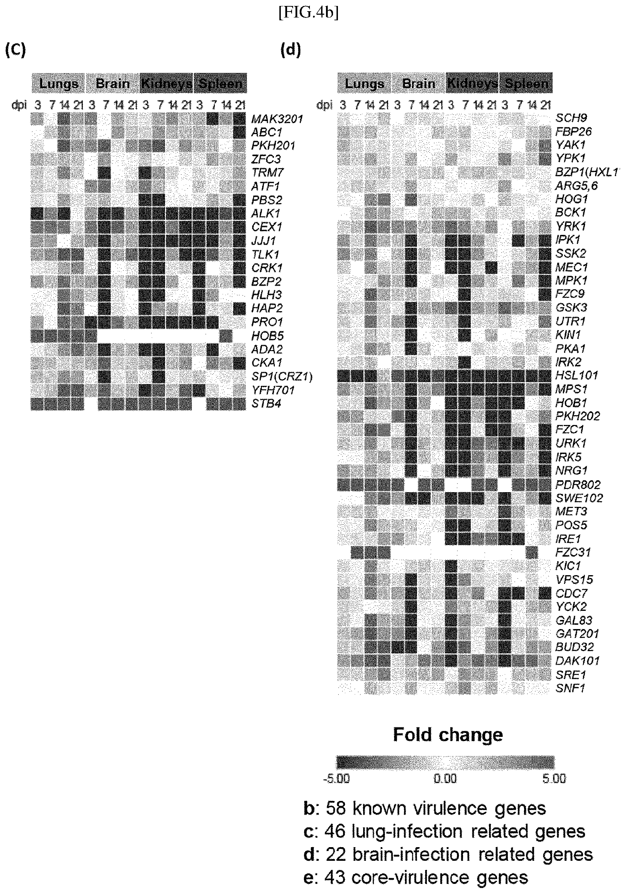

FIGS. 4A and 4B show a fold-change heatmap of gene expression of virulence-related genes, lung-virulence genes, brain-virulence genes and core virulence genes.
FIG. 5 shows the analysis results of passage through *in vitro* BBB using a transwell.
FIG. 6 shows the analysis results of whether the protein involved in passage through the brain-blood barrier adheres to endothelial cells and passes through the brain-blood barrier.
FIG. 7 shows NanoString clustering of proteins involved in passage through the brain-blood barrier.
FIG. 8 shows a schematic diagram in relation to ICV (intracerebroventricular) administration.
FIG. 9 shows the analysis results of proteins related to survival in the brain through ICV-STM scores.

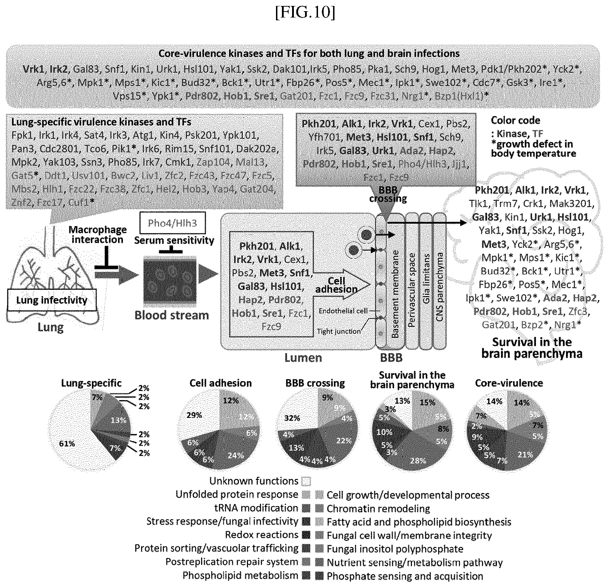

FIG. 10 shows a schematic diagram illustrating a brain infection-related signaling network.

### [Best Mode]

An aspect of the present invention relates to a method for screening an antifungal agent, in which the method includes: (a) contacting a sample to be analyzed with *Cryptococcus neoformans* cells containing an antifungal agent target protein; (b) measuring an amount or activity of the target protein; and (c) discriminating that the sample is an antifungal agent when it is measured that the amount or activity of the antifungal agent target protein is down-regulated in the prior stage, in which the target protein is a protein involved in passage through the BBB.

As used herein, the term "antifungal agent" is meant to include inorganic antifungal agents, organic natural extract-based antifungal agents, organic aliphatic compound-based antifungal agents, and organic aromatic compound-based antifungal agents, which serve to inhibit the propagation of eumycetes (fungi, yeast, and mushrooms). Examples of the inorganic antifungal agents include, but are not limited to, chlorine compounds (especially sodium hypochlorite), peroxides (especially hydrogen peroxide), boric acid compounds (especially boric acid and sodium borate), copper compounds (especially copper sulfate), zinc compounds (especially zinc sulfate and zinc chloride), sulfur-based compounds (especially sulfur, calcium polysulfate, and hydrated sulfur), calcium compounds (especially calcium oxide), silver compounds (especially thiosulfite silver complexes, and silver nitrate), in addition, iodine, sodium silicon fluoride, and the like. Examples of the organic natural extract-based antifungal agents include, but are not limited to, hinokitiol, Phyllostachys pubescens extracts, creosote oil, and the like.

The antifungal agent may be for treating or preventing meningoencephalitis or cryptococcosis, but is not limited thereto.

As used herein, the term "meningoencephalitis" refers to a disease including meningitis and/or encephalitis, which refers to an inflammatory disease of the brain parenchyma that occurs between the thin membrane surrounding the tissue and the brain or in the brain tissue.

The meningitis includes fungal meningoencephalitis, viral meningoencephalitis, tuberculous meningoencephalitis, and the like. In the case of fungal meningoencephalitis, it is infected through the respiratory tract and invades the central nervous system. Since eumycetes and mammals have evolutionarily similar cellular structures, it is difficult to discover targets for eumycetes only. Hence, fungal meningoencephalitis is one of the diseases that make it difficult to develop effective antifungal agents.

Based on cause, the encephalitis may be classified into infectious, vasculitis, neoplastic, chemical, idiopathic, etc., and may be classified into infectious encephalitis, tuberculous encephalitis, etc. with respect to the etiology. Encephalitis is one of the diseases with a high mortality rate of 70% to 80% if not treated according to early diagnosis.

"Cryptococcosis" of the present invention is an infectious disease caused by a yeast-type fungus called *Cryptococcus neoformans.* It mainly occurs in people with weakened immunity, and lung infections with respiratory symptoms may occur or the infection may spread to the central nervous system, leading to central nervous system infections such as meningitis or cryptococcosis.

As used herein, the team "sample" refers to an unknown candidate substance that is used in screening to examine whether it influences the expression level of a gene or the amount or activity of a protein. Examples of the sample include, but are not limited to, nucleic acids, peptides, polypeptides, chemical substances, and natural extracts.

In the present invention, the change in the amount or activity of a protein may be measured using two-dimensional electrophoresis, a biochip or an antibody that specifically binds to the protein. The biochip includes a protein chip, a nucleic acid array, or the like. In addition, the measurement method using the antibody that specifically binds to the protein may include a method selected from the group consisting of western blot, ELISA (enzyme-Linked Immunosorbent assay), colorimetric method, electrochemical method, fluorimetric method, luminometry, particle counting method, visual assessment and scintillation counting method, but is not limited thereto. It may be performed through a variety of known analytical methods.

As used herein, the term "brain-blood barrier (BBB)" refers to a barrier that separates cerebrospinal fluid from blood. Endothelial cells of brain capillaries form a tight junction to obstruct the movement of solutes between cells, thereby blocking the passage of polymers and hydrophilic substances.

As used herein, the term "protein involved in passage through the brain-blood barrier (BBB)" refers to all proteins such as kinases and transcription factors directly or indirectly involved in the passage of fungi such as eumycetes and/or bacteria through the brain-blood barrier.

In the present invention, a protein involved in passage and proliferation through the brain-blood barrier, which has not been revealed so far, among a series of infections caused by *Cryptococcus neoformans,* which causes meningoencephalitis, was investigated. In this regard, the present inventors identified a total of 23 proteins, including 14 kinases Cexl, Alkl, Pbs2, Yfh701, Pkh201, Met3, Hsl101, Snfl, Sch9, Irk5, Vrk1, Gal83, Urk1, and Irk2, and nine transcription factors Ada2, Hap2, Pho4/Hlh3, Srel, Fzc1, Pdr802, Fzc9, Hob1, and Jjj1, involved in adhesion and passage through the brain-blood barrier, and intended to provide a method for screening an antifungal agent using these proteins involved in passage through meninges.

Specifically, the protein involved in passage through the brain-blood barrier may be any one protein selected from the group consisting of Cexl, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1.

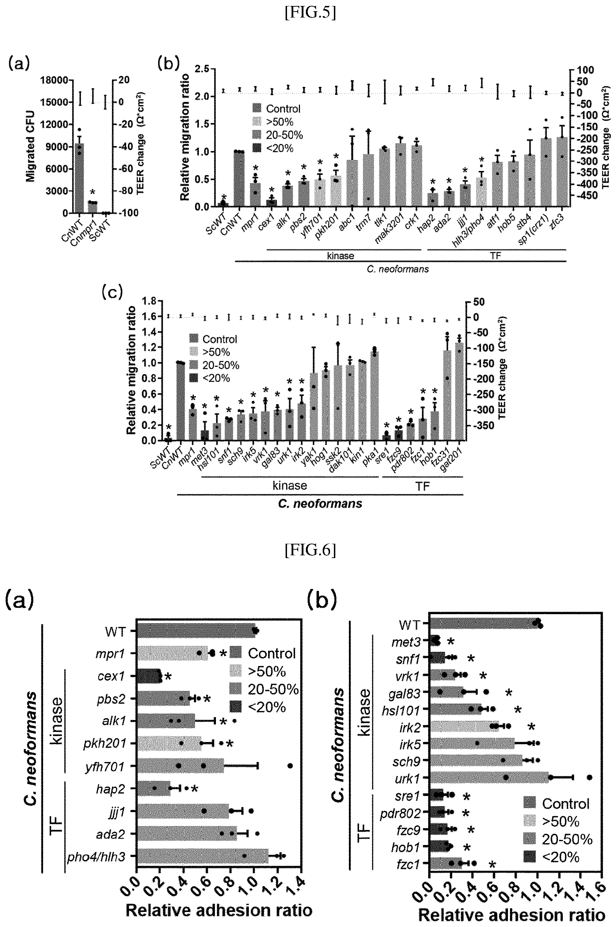

In one example of the present invention, as a result of identifying whether the brain-infection-related mutant transcription factors and kinases migrate through the brain-blood barrier through the *in vitro* BBB system, it was identified that 14 kinases Cexl, Alkl, Pbs2, Yfh701, Pkh201, Met3, Hsl101, Snfl, Sch9, Irk5, Vrk1, Gal83, Urk1, and Irk2, and nine transcription factors Ada2, Hap2, Pho4/Hlh3, Srel, Fzc1, Pdr802, Fzc9, Hob1, and Jjj1 were proteins required for passage through the brain-blood barrier (FIG. 5).

Specifically, any one protein selected from the group consisting of Cex1, Alkl, Pbs2, Pkh201, Met3, Hsl101, Snfl, Vrk1, Gal83, Irk2, Hap2, Srel, Fzc1, Pdr802, Fzc9 and Hob1 among the proteins involved in passage through the brain-blood barrier may be involved in adhesion through the brain-blood barrier.

In an example of the present invention, *Cryptococcus neoformans* first includes adhesion to the surface of endothelial cells in passage through the brain-blood barrier, and it was identified whether the protein involved in passage through the brain-blood barrier is also involved in the single molecule adhesion ability of endothelial cells, identifying that most proteins are also involved in the adhesion of endothelial cells. This suggests that adhesion to endothelial cells is one of the important pre-requisites for efficient passage of *Cryptococcus neoformans* through brain-blood barrier (FIG. 6).

In addition, specifically, any one protein selected from the group consisting of Alk1, Pkh201, Met3, Hsl101, Snfl, Gal83, Urk1, Irk2, Vrk1, Ada2, Hap2, Srel, Pdr802, and Hob1, which are proteins involved in passage through the brain-blood barrier, may also be a protein involved in survival inside the brain.

The "protein involved in survival inside the brain" includes all proteins that have proliferated *Cryptococcus neoformans* in the brain or that are necessary for and functionally related to the proliferation thereof.

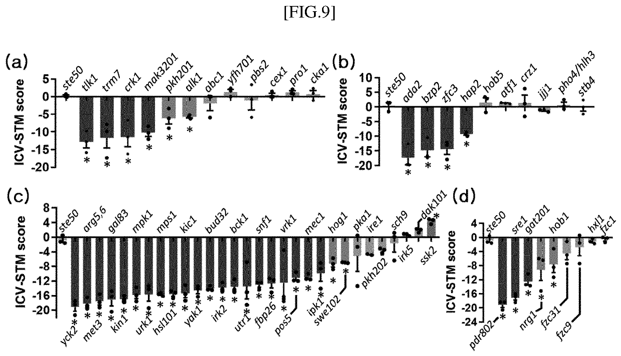

In one example of the present invention, in order to identify the protein involved in survival inside the brain, the mice were infected with the *Cryptococcus neoformans* strain, and then the infected brains were harvested and identified. As a result, 14 proteins of Alk1, Pkh201, Met3, Hsl101, Snfl, Gal83, Vrk1, Urk1, Irk2, Ada2, Hap2, Srel, Pdr802, and Hob1 were identified as proteins involved in both passage through the brain-blood barrier and survival inside the brain. In addition, it was identified that a total of 24 proteins, including 20 kinases (Tlk1, Trm7, Crk1, Mak3201, Yck2, Arg5/6, Kin1, Mpk1, Mps1, Kic1, Yak1, Bud32, Bck1, Utr1, Fpb26, Pos5, Mec1, Ipk1, Hog1, Swe102) and 4 transcription factors (Bzp2, Zfc3, Gat201, Nrg1), were not involved in passage through the brain-blood barrier, but were involved in proliferation of the *Cryptococcus neoformans* strain inside the brain (FIG. 9).

From the above results, it was identified that the *Cryptococcus neoformans* strain uses overlapping and distinct signaling pathways to be passed through the brain-blood barrier and proliferated inside the brain.

The proteins involved in passage through the brain-blood barrier and/or survival inside the brain may be involved in cell cycle regulation, tRNA migration, cell wall and membrane integrity, stress response and adaptation, lipid and sterol metabolism, vacuole transport, heme-mediated respiration control, ribosome biosynthesis, carbon utilization and gluconeogenesis, capsule biosynthesis, phosphate sensing and metabolism, and biological processes and signaling pathways such as Tor signaling.

For example, the biological functions and signaling pathways of proteins involved in passage through the brain-blood barrier include: Alk1 involved in cell cycle regulation; Cex1 involved in tRNA migration; Yfh701 involved in cell wall and membrane integrity; Pbs2 involved in stress response and adaptation; Pkh201, Sre1 and Hob1 involved in lipid and sterol metabolism; Hap2 involved in heme-mediated respiration control; Vrk1, Sch9 and Jjj 1 involved in ribosome biosynthesis; Pho4 involved in phosphate sensing and metabolism; and Sch9 involved in Tor signaling.

In addition, for example, the biological functions and signaling pathways of proteins involved in survival inside the brain include: Snfl, Gal83, Yck1, and Fpb26 involved in glucose sensing and metabolism; Kin1 involved in polarized exocytosis; Urk1 involved in pyrimidine ribonucleotide salvage pathway; Pos5 involved in mitochondrial function; Trm7 involved in tRNA modification; Mak3201 involved in replication or maintenance of double stranded RNA-containing particles; Crkl involved in meiosis activation (yeast Ime2 orthologous protein); Alkl, Swe102, Hsl101, and Ssn3 involved in cell cycle and morphology regulation; Pkh201 (yeast Pkh2 orthologous protein); Ada2 involved in histone acetyltransferase activity; Hap2 involved in heme-mediated respiration control; and Vrk1 involved in ribosome biogenesis.

Among these, two kinases including Pkh201 and Alk1 and four transcription factors including Hap2, Srel, Hob1, and Pdr802 were required for both passage and adhesion through the brain-blood barrier and survival inside the brain, indicating that lipid metabolism, cell cycle regulation, and heme-mediated respiration control are critical for both passage and adhesion through the brain-blood barrier and survival inside the brain.

In addition, specifically, stages (a) and (b) in the method for screening an antifungal agent may be performed between 30°C and 40°C.

Another aspect of the present invention relates to a method for screening an antifungal agent for co-administration, in which the method includes: (a) a first measurement stage of contacting an antifungal agent with *Cryptococcus neoformans* cells containing an antifungal agent target protein, and measuring an amount or activity of the protein; (b) a second measurement stage of contacting a sample to be analyzed and the antifungal agent with *Cryptococcus neoformans* cells containing an antifungal agent target protein, and measuring an amount or activity of the protein; and (c) comparing measured values of the first and second measurement stages, and when the measured value of the second measurement stage is down-regulated from the measured value of the first measurement stage, discriminating that the sample is an antifungal agent for co-administration, in which the target protein is a protein involved in passage through the brain-blood barrier.

As used herein, the term "co-administration" refers to a case in which the effect is increased when several drugs are administered together, compared to when administered alone. In the present invention, the term refers to a case in which the antifungal effect is increased when the screened antifungal agent is administered together than when the conventional known antifungal agent is administered alone.

The protein involved in passage through the brain-blood barrier may be any one selected from the group consisting of Cex1, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1.

Specifically, any one protein selected from the group consisting of Cex1, Alk1, Pbs2, Pkh201, Met3, Hsl101, Snfl, Vrk1, Gal83, Irk2, Hap2, Srel, Fzc1, Pdr802, Fzc9 and Hob1 among the proteins involved in passage through the brain-blood barrier may be involved in adhesion through the brain-blood barrier.

In addition, specifically, any one protein selected from the group consisting of 14 proteins of Alk1, Pkh201, Met3, Hsl101, Snfl, Gal83, Urk1, Irk2, Vrk1, Ada2, Hap2, Srel, Pdr802 and Hob1 among the proteins involved in passage through the brain-blood barrier may also be a protein involved in survival inside the brain.

In addition, specifically, the antifungal agent in stage (a) may be an azole-based or non-azole-based antifungal agent.

More specifically, the azole-based antifungal agent may be any one or more of fluconazole, itraconazole, voriconazole, and ketoconazole.

In addition, the non-azole-based antifungal agent may be amphotericin B or fludioxonil.

The antifungal agent for co-administration may be for treating meningoencephalitis or cryptococcosis.

Yet another aspect of the present invention relates to a biomarker composition for diagnosing meningoencephalitis or cryptococcosis, in which the composition includes any one or more proteins involved in passage through the brain-blood barrier selected from the group consisting of Cex1, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1.

The descriptions of "meningoencephalitis" or "cryptococcosis" are the same as described above.

As used herein, the term "biomarker for diagnosing meningoencephalitis or cryptococcosis" is designed to diagnose whether it is meningoencephalitis or cryptococcosis using a difference in the amount or activity level of proteins involved in passage through the brain-blood barrier.

As used herein, the term "diagnosis" means determining meningoencephalitis or cryptococcosis, and specifically, it may be determined by comparing whether the level of the amount or activity of proteins involved in passage through the brain-blood barrier is high or low.

Specifically, when an amount or activity of any one or more proteins among the proteins involved in passage through the brain-blood barrier is down-regulated, it may be diagnosed with meningoencephalitis or cryptococcosis.

For example, when an amount or activity level of any one or more proteins selected from the group consisting of Cex1, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1, which are proteins involved in passage through the brain-blood barrier is measured and down-regulated, it may be diagnosed with meningoencephalitis or cryptococcosis.

The biomarker composition may include an agent capable of measuring the level of a protein involved in passage through the brain-blood barrier, and the agent may measure the protein amount or activity of each of the 23 proteins involved in passage through the brain-blood barrier.

Another aspect of the present invention relates to a kit for diagnosing meningoencephalitis or cryptococcosis, in which the kit includes a biomarker composition for diagnosing meningoencephalitis or cryptococcosis.

The kit may include, without limitation, an agent capable of measuring the amount or activity of a protein involved in passage through the brain-blood barrier, other components, solutions or devices used for measurement, and the like, and instructions for use of the kit may be added.

The kit may include a conventional well-shaped microtiter plate capable of holding a sample. The well may include a porous support capable of adsorbing the sample and one or more biomarkers, which are known in the conventional technical field and are commercially available.

Yet another aspect of the present invention relates to an antifungal pharmaceutical composition including an inhibitor for one or more proteins involved in passage through the brain-blood barrier selected from the group consisting of Cexl, Alkl, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1 of *Cryptococcus neoformans.*

The descriptions of "meningoencephalitis" or "cryptococcosis" are the same as described above.

As used herein, the term "inhibitor" refers to a nucleic acid, a peptide, a polypeptide, a chemical or natural substance that degrades or inhibits the expression or activity of kinases or transcription factors directly or indirectly involved in endothelial cell adhesion when passing through the brain-blood barrier or prior to passing the brain-blood barrier.

Specifically, the inhibitor may be any one or more of an antibody, a dominant-negative mutation, and a ribozyme against the protein involved in passage through the brain-blood barrier (BBB).

In addition, specifically, the inhibitor may be an antisense oligonucleotide, siRNA, shRNA, miRNA, or a vector containing the same for a gene encoding the protein involved in passage through the brain-blood barrier (BBB), but is not limited thereto. All inhibitors capable of inhibiting expression of the gene are included.

Yet another aspect of the present invention relates to a pharmaceutical composition for treating or preventing meningoencephalitis or cryptococcosis, in which the pharmaceutical composition includes the antifungal pharmaceutical composition.

The descriptions of "antifungal pharmaceutical composition," "meningoencephalitis" or "cryptococcosis" are the same as described above.

The pharmaceutical composition according to the present invention may be prepared into a pharmaceutical formulation using methods well known in the pertinent field to provide rapid, sustained or delayed release of an active ingredient after administration to a mammal. In the preparation of the formulation, the pharmaceutical composition according to the present invention may additionally include a pharmaceutically acceptable carrier within a range that does not inhibit an activity of a compound of the present invention.

The pharmaceutically acceptable carrier includes, but is not limited thereto, conventionally used ones, for example, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propylhydroxybenzoate, talc, magnesium stearate, mineral oil, and the like. In addition, the pharmaceutical composition of the present invention may include a diluent or an excipient such as filler, extender, binder, humectant, disintegrant, and surfactant, and other pharmaceutically acceptable additives.

An administration of the pharmaceutical composition according to the present invention needs to be a pharmaceutically effective amount. The "pharmaceutically effective amount" means an amount enough to prevent or treat diseases at a reasonable benefit/risk ratio applicable to medical treatment. An effective dose level may be variously selected by those skilled in the art according to such factors as a formulation method, a patient's condition and weight, the patient's gender, age and degree of disease, a drug form, an administration route and period, an excretion rate, reaction sensitivity, etc. The effective amount may vary depending on a route of treatment, a use of excipient and a possibility of being used with other drugs, as recognized by those skilled in the art. However, in case of a preparation for oral administration to achieve a preferable effect, the composition according to the present invention may be generally administered to an adult in an amount of 0.0001 to 100 mg/kg a day, preferably 0.001 to 100 mg/kg a day based on 1 kg of body weight. However, the dosage does not limit the scope of the present invention in any aspect.

The pharmaceutical composition of the present invention may be administered to mammals such as mice, livestock, and humans, through various routes. Specifically, the pharmaceutical composition according to the present invention may be orally or parenterally administered (for example, applied or injected intravenously, subcutaneously or intraperitoneally), but may be preferably orally administered. For the prevention and treatment of vaginitis, it may be administered intravaginally. A solid preparation for oral administration may include powder, granule, tablet, capsule, soft capsule, pill, etc. A liquid preparation for oral administration may include a suspending agent, liquid for internal use, emulsion, syrup, aerosol, etc., but may also include various excipients, for example, humectant, a sweetening agent, a flavoring agent, preservative, etc. in addition to water and liquid paraffin, which are frequently used simple diluents. A preparation for parenteral administration may be used by being formulated into a dosage form of external preparation and sterilized injectable preparation such as sterilized aqueous solution, liquid, non-aqueous solvent, a suspending agent, emulsion, eye drop, eye ointment, syrup, suppository, and aerosol, according to respective conventional methods, and preferably may be used by preparing a pharmaceutical composition of cream, gel, patch, spray, ointment, plaster, lotion, liniment, eye ointment, eye drop, paste or cataplasma, but not limited thereto. A preparation for local administration may be an anhydrous or aqueous form depending on a clinical prescription. As the non-aqueous solvent and the suspending agent above, propylene glycol, polyethylene glycol, vegetable oil like olive oil, injectable ester like ethyl oleate, etc. may be used. As a base of the suppository above, witepsol, macrogol, tween 61, cacao butter, laurin oil, glycerogelatin, etc. may be used.

Yet another aspect of the present invention provides a method for treating meningoencephalitis or cryptococcosis, in which the method includes administering to a subject the pharmaceutical composition for treating or preventing meningoencephalitis or cryptococcosis.

The terms "meningoencephalitis" and "cryptococcosis" according to the present invention are the same as described above.

The subject refers to an animal, and may be typically a mammal, on which treatment using the compound of the present invention may show a beneficial effect. A preferable example of such subject may include primates like a human being. In addition, such subjects may include all the subjects having a symptom of diabetes, or having a risk of having such symptom.

### [Mode]

Hereinafter, the present invention will be described in detail by way of the examples. However, the following examples are provided only for the purpose of illustrating the present invention, and thus the present invention is not limited thereto.

### Example 1. Strain and culture conditions

In the present invention, strains used to analyze transcription factors and kinases related to meningoencephalitis induction are shown in Table 1 below. *Cryptococcus neoformans* was cultured in a yeast extract-peptone dextrose (YPD) medium unless otherwise indicated.

**[Table 1]**

| **No.** | **Strain** | **Genotype** | **Parent** | **Accession No. / Source** |
|---|---|---|---|---|
| 1 | H99 | *MAT*α | - | 208821 / ATCC |
| 2 | YSB3741 | *MAT*α *ste50*Δ:: *NAT-STM#234* | H99 | 51297 / KCCM |
| 3 | YSB2344 | *MAT*α *ste50*Δ:: *NAT-STM#282* | H99 | 51291 / KCCM |
| 4 | YSB2492 | *MAT*α *ire1*Δ:: *NAT-STM#169* | H99 | 51297 / KCCM |
| 5 | XL280 | *MAT*α | - | those possessed by Duke University / Duke University |
| 6 | R265 | *MAT*α | - | MYA-4093 / ATCC |
| 7 | BY4742 | *Saccharomyces cerevisiae MAT*α | - | 201389 / ATCC |
| 8 | YSB5492 | *MAT*α *mpr1*Δ:: *NAT-STM#116* | H99 | those possessed by Yonsei University / Yonsei University |
| 9 | YSB4211 | *MAT*α *adk1*Δ:: *NAT-STM#43* | H99 | 51297 / KCCM |
| 10 | YSB4212 | *MAT*α *adk1*Δ:: *NAT-STM#43* | H99 | 51297 / KCCM |
| 11 | YSB 1503 | *MAT*α *arg2*Δ:: *NAT-STM#125* | H99 | 51297 / KCCM |
| 12 | YSB 1504 | *MAT*α *arg2*Δ:: *NAT-STM#125* | H99 | 51297 / KCCM |
| 13 | YSB2408 | *MAT*α *arg5,6*Δ:: *NAT-STM#220* | H99 | 51297 / KCCM |
| 14 | YSB2409 | *MAT*α *arg5,6*Δ:: *NAT-STM#220* | H99 | 51297 / KCCM |
| 15 | YSB 1725 | *MAT*α *ark1*Δ:: *NAT-STM#43* | H99 | 51297 / KCCM |
| 16 | YSB 1726 | *MAT*α *ark1*Δ:: *NAT-STM#43* | H99 | 51297 / KCCM |
| 17 | YSB 1935 | *MAT*α *atg1*Δ:: *NAT-STM#288* | H99 | 51297 / KCCM |
| 18 | YSB 1936 | *MAT*α *atg1*Δ:: *NAT-STM#288* | H99 | 51297 / KCCM |
| 19 | YSB273 | *MAT*α *bck1*Δ:: *NAT-STM#43* | H99 | 51297 / KCCM |
| 20 | YSB274 | *MAT*α *bck1*Δ:: *NAT-STM#43* | H99 | 51297 / KCCM |
| 21 | YSB4190 | *MAT*α *bub1*Δ:: *NAT-STM#201* | H99 | 51297 / KCCM |
| 22 | YSB4191 | *MAT*α *bub1*Δ:: *NAT-STM#201* | H99 | 51297 / KCCM |
| 23 | YSB4336 | *MAT*α *bud16*Δ:: *NAT-STM#232* | H99 | 51297 / KCCM |
| 24 | YSB4337 | *MAT*α *bud16*Δ:: *NAT-STM#232* | H99 | 51297 / KCCM |
| 25 | YSB 1968 | *MAT*α *bud32*Δ:: *NAT-STM#295* | H99 | 51297 / KCCM |
| 26 | YSB 1969 | *MAT*α *bud32*Δ:: *NAT-STM#295* | H99 | 51297 / KCCM |
| 27 | YSB2941 | *MAT*α *cbk1*Δ:: *NAT-STM#232* | H99 | 51297 / KCCM |
| 28 | YSB2942 | *MAT*α *cbk1*Δ:: *NAT-STM#232* | H99 | 51297 / KCCM |
| 29 | YSB2370 | *MAT*α *cdc2801*Δ:: *NAT-STM#191* | H99 | 51297 / KCCM |
| 30 | YSB3699 | *MAT*α *cdc2801*Δ:: *NAT-STM#191* | H99 | 51297 / KCCM |
| 31 | YSB2911 | *MAT*α *cdc7*Δ:: *NAT-STM#213* | H99 | 51297 / KCCM |
| 32 | YSB2912 | *MAT*α *cdc7*Δ:: *NAT-STM#213* | H99 | 51297 / KCCM |
| 33 | YSB 1825 | *MAT*α *chk1*Δ:: *NAT-STM#205* | H99 | 51297 / KCCM |
| 34 | YSB 1828 | *MAT*α *chk1*Δ:: *NAT-STM#205* | H99 | 51297 / KCCM |
| 35 | YSB3051 | *MAT*α *cka1*Δ:: *NAT-STM#6* | H99 | 51297 / KCCM |
| 36 | YSB3052 | *MAT*α *cka1*Δ:: *NAT-STM#6* | H99 | 51297 / KCCM |
| 37 | YSB 1804 | *MAT*α *cki1*Δ:: *NAT-STM#218* | H99 | 51297 / KCCM |
| 38 | YSB 1805 | *MAT*α *cki1*Δ:: *NAT-STM#218* | H99 | 51297 / KCCM |
| 39 | YSB 1245 | *MAT*α *cky1*Δ:: *NAT-STM#282* | H99 | 51297 / KCCM |
| 40 | YSB 1246 | *MAT*α *cky1*Δ:: *NAT-STM#282* | H99 | 51297 / KCCM |
| 41 | YSB 1883 | *MAT*α *cmk1*Δ:: *NAT-STM#227* | H99 | 51297 / KCCM |
| 42 | YSB 1901 | *MAT*α *cmk1*Δ:: *NAT-STM#227* | H99 | 51297 / KCCM |
| 43 | YSB4256 | *MAT*α *cmk2*Δ:: *NAT-STM#220* | H99 | 51297 / KCCM |
| 44 | YSB4257 | *MAT*α *cmk2*Δ:: *NAT-STM#220* | H99 | 51297 / KCCM |
| 45 | YSB 127 | *MAT*α *cpk1*Δ:: *NAT-STM#184* | H99 | 51297 / KCCM |
| 46 | YSB 128 | *MAT*α *cpk1*Δ:: *NAT-STM#184* | H99 | 51297 / KCCM |
| 47 | YSB373 | *MAT*α *cpk2*Δ:: *NAT-STM#122* | H99 | 51297 / KCCM |
| 48 | YSB374 | *MAT*α *cpk2*Δ:: *NAT-STM#122* | H99 | 51297 / KCCM |
| 49 | YSB 1940 | *MAT*α *dak102*Δ:: *NAT-STM#295* | H99 | 51297 / KCCM |
| 50 | YSB 1941 | *MAT*α *dak102*Δ:: *NAT-STM#295* | H99 | 51297 / KCCM |
| 51 | YSB2487 | *MAT*α *dak202a*Δ:: *NAT-STM#119* | H99 | 51297 / KCCM |
| 52 | YSB2489 | *MAT*α *dak202a*Δ:: *NAT-STM#119* | H99 | 51297 / KCCM |
| 53 | YSB4252 | *MAT*α *env7*Δ:: *NAT-STM#227* | H99 | 51297 / KCCM |
| 54 | YSB4253 | *MAT*α *env7*Δ:: *NAT-STM#227* | H99 | 51297 / KCCM |
| 55 | YSB3172 | *MAT*α *fab1*Δ:: *NAT-STM#169* | H99 | 51297 / KCCM |
| 56 | YSB4281 | *MAT*α *fab1*Δ:: *NAT-STM#169* | H99 | 51297 / KCCM |
| 57 | YSB4341 | *MAT*α *fbp26*Δ:: *NAT-STM#146* | H99 | 51297 / KCCM |
| 58 | YSB4342 | *MAT*α *fbp26*Δ:: *NAT-STM#146* | H99 | 51297 / KCCM |
| 59 | YSB2948 | *MAT*α *fpk1*Δ:: *NAT-STM#211* | H99 | 51297 / KCCM |
| 60 | YSB2949 | *MAT*α *fpk1*Δ:: *NAT-STM#211* | H99 | 51297 / KCCM |
| 61 | YSB4228 | *MAT*α *frk101*Δ:: *NAT-STM#282* | H99 | 51297 / KCCM |
| 62 | YSB4230 | *MAT*α *frk101*Δ:: *NAT-STM#282* | H99 | 51297 / KCCM |
| 63 | YSB4324 | *MAT*α *frk102*Δ:: *NAT-STM#231* | H99 | 51297 / KCCM |
| 64 | YSB4325 | *MAT*α *frk102*Δ:: *NAT-STM#231* | H99 | 51297 / KCCM |
| 65 | YSB2829 | *MAT*α *gal1*Δ:: *NAT-STM#224* | H99 | 51297 / KCCM |
| 66 | YSB2830 | *MAT*α *gal1*Δ:: *NAT-STM#224* | H99 | 51297 / KCCM |
| 67 | YSB2852 | *MAT*α *gal302*Δ:: *NAT-STM#218* | H99 | 51297 / KCCM |
| 68 | YSB2853 | *MAT*α *gal302*Δ:: *NAT-STM#218* | H99 | 51297 / KCCM |
| 69 | YSB2038 | *MAT*α *gsk3*Δ:: *NAT-STM#123* | H99 | 51297 / KCCM |
| 70 | YSB2039 | *MAT*α *gsk3*Δ:: *NAT-STM#123* | H99 | 51297 / KCCM |
| 71 | YSB 1241 | *MAT*α *gut1*Δ:: *NAT-STM#242* | H99 | 51297 / KCCM |
| 72 | YSB2761 | *MAT*α *gut1*Δ:: *NAT-STM#242* | H99 | 51297 / KCCM |
| 73 | YSB1438 | *MAT*α *hrk1*/*mph1*Δ:: *NAT-STM#210* | H99 | 51297 / KCCM |
| 74 | YSB 1439 | *MAT*α *hrk1*/*mph1*Δ:: *NAT-STM#210* | H99 | 51297 / KCCM |
| 75 | YSB270 | *MAT*α *hrk1*Δ:: *NAT-STM#58* | H99 | 51297 / KCCM |
| 76 | YSB271 | *MAT*α *hrk1*Δ:: *NAT-STM#58* | H99 | 51297 / KCCM |
| 77 | YSB4174 | *MAT*α *hrr2502*Δ:: *NAT-STM#125* | H99 | 51297 / KCCM |
| 78 | YSB4176 | *MAT*α *hrr2502*Δ:: *NAT-STM#125* | H99 | 51297 / KCCM |
| 79 | YSB1514 | *MAT*α *igi1*Δ:: *NAT-STM#230* | H99 | 51297 / KCCM |
| 80 | YSB1515 | *MAT*α *igi1*Δ:: *NAT-STM#230* | H99 | 51297 / KCCM |
| 81 | YSB 1310 | *MAT*α *iks1*Δ:: *NAT-STM#116* | H99 | 51297 / KCCM |
| 82 | YSB2119 | *MAT*α *iks1*Δ:: *NAT-STM#116* | H99 | 51297 / KCCM |
| 83 | YSB2157 | *MAT*α *ipk1*Δ:: *NAT-STM#184* | H99 | 51297 / KCCM |
| 84 | YSB2158 | *MAT*α *ipk1*Δ:: *NAT-STM#184* | H99 | 51297 / KCCM |
| 85 | YSB552 | *MAT*α *ire1*Δ:: *NAT-STM#224* | H99 | 51297 / KCCM |
| 86 | YSB554 | *MAT*α *ire1*Δ:: *NAT-STM#224* | H99 | 51297 / KCCM |
| 87 | YSB 1950 | *MAT*α *irk1*Δ:: *NAT-STM#5* | H99 | 51297 / KCCM |
| 88 | YSB 1951 | *MAT*α *irk1*Δ:: *NAT-STM#5* | H99 | 51297 / KCCM |
| 89 | YSB 1486 | *MAT*α *irk3*Δ:: *NAT-STM#273* | H99 | 51297 / KCCM |
| 90 | YSB 1487 | *MAT*α *irk3*Δ:: *NAT-STM#273* | H99 | 51297 / KCCM |
| 91 | YSB2806 | *MAT*α *irk4*Δ:: *NAT-STM#211* | H99 | 51297 / KCCM |
| 92 | YSB2808 | *MAT*α *irk4*Δ:: *NAT-STM#211* | H99 | 51297 / KCCM |
| 93 | YSB3830 | *MAT*α *irk6*Δ:: *NAT-STM#5* | H99 | 51297 / KCCM |
| 94 | YSB3831 | *MAT*α *irk6*Δ:: *NAT-STM#5* | H99 | 51297 / KCCM |
| 95 | YSB2136 | *MAT*α *irk7*Δ:: *NAT-STM#208* | H99 | 51297 / KCCM |
| 96 | YSB2137 | *MAT*α *irk7*Δ:: *NAT-STM#208* | H99 | 51297 / KCCM |
| 97 | YSB3211 | *MAT*α *kic102*Δ:: *NAT-STM#201* | H99 | 51297 / KCCM |
| 98 | YSB3212 | *MAT*α *kic102*Δ:: *NAT-STM#201* | H99 | 51297 / KCCM |
| 99 | YSB2915 | *MAT*α *kic1*Δ:: *NAT-STM#201* | H99 | 51297 / KCCM |
| 100 | YSB2916 | *MAT*α *kic1*Δ:: *NAT-STM#201* | H99 | 51297 / KCCM |
| 101 | YSB2955 | *MAT*α *kin4*Δ:: *NAT-STM#225* | H99 | 51297 / KCCM |
| 102 | YSB4156 | *MAT*α *kin4*Δ:: *NAT-STM#225* | H99 | 51297 / KCCM |
| 103 | YSB 1807 | *MAT*α *ksp1*Δ:: *NAT-STM#159* | H99 | 51297 / KCCM |
| 104 | YSB 1808 | *MAT*α *ksp1*Δ:: *NAT-STM#159* | H99 | 51297 / KCCM |
| 105 | YSB3789 | *MAT*α *lcb5*Δ:: *NAT-STM#213* | H99 | 51297 / KCCM |
| 106 | YSB3790 | *MAT*α *lcb5*Δ:: *NAT-STM#213* | H99 | 51297 / KCCM |
| 107 | YSB3240 | *MAT*α *mak3202*Δ:: *NAT-STM#169* | H99 | 51297 / KCCM |
| 108 | YSB3241 | *MAT*α *mak3202*Δ:: *NAT-STM#169* | H99 | 51297 / KCCM |
| 109 | YSB3063 | *MAT*α *mec1*Δ:: *NAT-STM#204* | H99 | 51297 / KCCM |
| 110 | YSB3611 | *MAT*α *mec1*Δ:: *NAT-STM#204* | H99 | 51297 / KCCM |
| 111 | YSB330 | *MAT*α *mkk2*Δ:: *NAT-STM#224* | H99 | 51297 / KCCM |
| 112 | YSB331 | *MAT*α *mkk2*Δ:: *NAT-STM#224* | H99 | 51297 / KCCM |
| 113 | YSB3814 | *MAT*α *mpk1*Δ:: *NAT-STM#240* | H99 | 51297 / KCCM |
| 114 | YSB3816 | *MAT*α *mpk1*Δ:: *NAT-STM#240* | H99 | 51297 / KCCM |
| 115 | YSB3236 | *MAT*α *mpk2*Δ:: *NAT-STM#102* | H99 | 51297 / KCCM |
| 116 | YSB3238 | *MAT*α *mpk2*Δ:: *NAT-STM#102* | H99 | 51297 / KCCM |
| 117 | YSB3632 | *MAT*α *mps1*Δ:: *NAT-STM#116* | H99 | 51297 / KCCM |
| 118 | YSB3633 | *MAT*α *mps1*Δ:: *NAT-STM#116* | H99 | 51297 / KCCM |
| 119 | YSB4288 | *MAT*α *oxk1*Δ:: *NAT-STM#122* | H99 | 51297 / KCCM |
| 120 | YSB4289 | *MAT*α *oxk1*Δ:: *NAT-STM#122* | H99 | 51297 / KCCM |
| 121 | YSB2809 | *MAT*α *pan3*Δ:: *NAT-STM#204* | H99 | 51297 / KCCM |
| 122 | YSB2810 | *MAT*α *pan3*Δ:: *NAT-STM#204* | H99 | 51297 / KCCM |
| 123 | YSB4338 | *MAT*α *pho8501*Δ:: *NAT-STM#273* | H99 | 51297 / KCCM |
| 124 | YSB4339 | *MAT*α *pho8501*Δ:: *NAT-STM#273* | H99 | 51297 / KCCM |
| 125 | YSB3702 | *MAT*α *pho85*Δ:: *NAT-STM#218* | H99 | 51297 / KCCM |
| 126 | YSB3703 | *MAT*α *pho85*Δ:: *NAT-STM#218* | H99 | 51297 / KCCM |
| 127 | YSB1493 | *MAT*α *pik1*Δ:: *NAT-STM#227* | H99 | 51297 / KCCM |
| 128 | YSB1494 | *MAT*α *pik1*Δ:: *NAT-STM#227* | H99 | 51297 / KCCM |
| 129 | YSB194 | *MAT*α *pka2*Δ:: *NAT-STM#205* | H99 | 51297 / KCCM |
| 130 | YSB195 | *MAT*α *pka2*Δ:: *NAT-STM#205* | H99 | 51297 / KCCM |
| 131 | YSB4268 | *MAT*α *pkh202*Δ:: *NAT-STM#218* | H99 | 51297 / KCCM |
| 132 | YSB4309 | *MAT*α *pkh202*Δ:: *NAT-STM#218* | H99 | 51297 / KCCM |
| 133 | YSB558 | *MAT*α *pkp1*Δ:: *NAT-STM#224* | H99 | 51297 / KCCM |
| 134 | YSB608 | *MAT*α *pkp1*Δ:: *NAT-STM#224* | H99 | 51297 / KCCM |
| 135 | YSB2439 | *MAT*α *pkp2*Δ:: *NAT-STM#295* | H99 | 51297 / KCCM |
| 136 | YSB2440 | *MAT*α *pkp2*Δ:: *NAT-STM#295* | H99 | 51297 / KCCM |
| 137 | YSB3714 | *MAT*α *pos5*Δ:: *NAT-STM#58* | H99 | 51297 / KCCM |
| 138 | YSB3715 | *MAT*α *pos5*Δ:: *NAT-STM#58* | H99 | 51297 / KCCM |
| 139 | YSB4269 | *MAT*α *pro1*Δ:: *NAT-STM#5* | H99 | 51297 / KCCM |
| 140 | YSB4270 | *MAT*α *pro1*Δ:: *NAT-STM#5* | H99 | 51297 / KCCM |
| 141 | YSB 1989 | *MAT*α *psk201*Δ:: *NAT-STM#191* | H99 | 51297 / KCCM |
| 142 | YSB 1990 | *MATα psk201*Δ:: *NAT-STM#191* | H99 | 51297 / KCCM |
| 143 | YSB2443 | *MAT*α *psk202*Δ:: *NAT-STM#208* | H99 | 51297 / KCCM |
| 144 | YSB2444 | *MAT*α *psk202*Δ:: *NAT-STM#208* | H99 | 51297 / KCCM |
| 145 | YSB3785 | *MAT*α *rad53*Δ:: *NAT-STM#184* | H99 | 51297 / KCCM |
| 146 | YSB3786 | *MAT*α *rad53*Δ:: *NAT-STM#184* | H99 | 51297 / KCCM |
| 147 | YSB1510 | *MAT*α *rbk1*Δ:: *NAT-STM#219* | H99 | 51297 / KCCM |
| 148 | YSB1511 | *MAT*α *rbk1*Δ:: *NAT-STM#219* | H99 | 51297 / KCCM |
| 149 | YSB1579 | *MAT*α *rik1*Δ:: *NAT-STM#150* | H99 | 51297 / KCCM |
| 150 | YSB1580 | *MAT*α *rik1*Δ:: *NAT-STM#150* | H99 | 51297 / KCCM |
| 151 | YSB 1216 | *MAT*α *rim15*Δ:: *NAT-STM#191* | H99 | 51297 / KCCM |
| 152 | YSB 1217 | *MAT*α *rim15*Δ:: *NAT-STM#191* | H99 | 51297 / KCCM |
| 153 | YSB4347 | *MAT*α *sat4*Δ:: *NAT-STM#212* | H99 | 51297 / KCCM |
| 154 | YSB4348 | *MAT*α *sat4*Δ:: *NAT-STM#212* | H99 | 51297 / KCCM |
| 155 | YSB2793 | *MAT*α *scy1*Δ:: *NAT-STM#150* | H99 | 51297 / KCCM |
| 156 | YSB2794 | *MAT*α *scy1*Δ:: *NAT-STM#150* | H99 | 51297 / KCCM |
| 157 | YSB1410 | *MAT*α *sks1*Δ:: *NAT-STM#211* | H99 | 51297 / KCCM |
| 158 | YSB 1411 | *MAT*α *sks1*Δ:: *NAT-STM#211* | H99 | 51297 / KCCM |
| 159 | YSB1575 | *MAT*α *snf101*Δ:: *NAT-STM#146* | H99 | 51297 / KCCM |
| 160 | YSB1576 | *MAT*α *snf101*Δ:: *NAT-STM#146* | H99 | 51297 / KCCM |
| 161 | YSB4321 | *MAT*α *snf102*Δ:: *NAT-STM#116* | H99 | 51297 / KCCM |
| 162 | YSB4323 | *MAT*α *snf102*Δ:: *NAT-STM#116* | H99 | 51297 / KCCM |
| 163 | YSB3229 | *MAT*α *sps1*Δ:: *NAT-STM#288* | H99 | 51297 / KCCM |
| 164 | YSB3325 | *MAT*α *sps1*Δ:: *NAT-STM#288* | H99 | 51297 / KCCM |
| 165 | YSB3038 | *MAT*α *ssn3*Δ:: *NAT-STM#219* | H99 | 51297 / KCCM |
| 166 | YSB3039 | *MAT*α *ssn3*Δ:: *NAT-STM#219* | H99 | 51297 / KCCM |
| 167 | YSB313 | *MAT*α *ste11*Δ:: *NAT-STM#242* | H99 | 51297 / KCCM |
| 168 | YSB314 | *MAT*α *ste11*Δ:: *NAT-STM#242* | H99 | 51297 / KCCM |
| 169 | YSB342 | *MAT*α *ste7*Δ:: *NAT-STM#225* | H99 | 51297 / KCCM |
| 170 | YSB343 | *MAT*α *ste7*Δ:: *NAT-STM#225* | H99 | 51297 / KCCM |
| 171 | YSB1564 | *MAT*α *swe102*Δ:: *NAT-STM#169* | H99 | 51297 / KCCM |
| 172 | YSB1565 | *MAT*α *swe102*Δ:: *NAT-STM#169* | H99 | 51297 / KCCM |
| 173 | YSB278 | *MAT*α *tco1*Δ:: *NAT-STM#102* | H99 | 51297 / KCCM |
| 174 | YSB279 | *MAT*α *tco1*Δ:: *NAT-STM#102* | H99 | 51297 / KCCM |
| 175 | YSB281 | *MAT*α *tco2*Δ:: *NAT-STM#116* | H99 | 51297 / KCCM |
| 176 | YSB282 | *MAT*α *tco2*Δ:: *NAT-STM#116* | H99 | 51297 / KCCM |
| 177 | YSB284 | *MAT*α *tco3*Δ:: *NAT-STM#119* | H99 | 51297 / KCCM |
| 178 | YSB285 | *MAT*α *tco3*Δ:: *NAT-STM#119* | H99 | 51297 / KCCM |
| 179 | YSB417 | *MAT*α *tco4*Δ:: *NAT-STM#123* | H99 | 51297 / KCCM |
| 180 | YSB418 | *MAT*α *tco4*Δ:: *NAT-STM#123* | H99 | 51297 / KCCM |
| 181 | YSB286 | *MAT*α *tco5*Δ:: *NAT-STM#125* | H99 | 51297 / KCCM |
| 182 | YSB287 | *MAT*α *tco5*Δ:: *NAT-STM#125* | H99 | 51297 / KCCM |
| 183 | YSB2469 | *MAT*α *tco6*Δ:: *NAT-STM#58* | H99 | 51297 / KCCM |
| 184 | YSB2554 | *MAT*α *tco6*Δ:: *NAT-STM#58* | H99 | 51297 / KCCM |
| 185 | YSB4186 | *MAT*α *tco7*Δ:: *NAT-STM#208* | H99 | 51297 / KCCM |
| 186 | YSB4187 | *MAT*α *tco7*Δ:: *NAT-STM#208* | H99 | 51297 / KCCM |
| 187 | YSB2663 | *MAT*α *tda10*Δ:: *NAT-STM#102* | H99 | 51297 / KCCM |
| 188 | YSB3223 | *MAT*α *tda10*Δ:: *NAT-STM#102* | H99 | 51297 / KCCM |
| 189 | YSB3844 | *MAT*α *tel1*Δ:: *NAT-STM#225* | H99 | 51297 / KCCM |
| 190 | YSB3845 | *MAT*α *tel1*Δ:: *NAT-STM#225* | H99 | 51297 / KCCM |
| 191 | YSB3219 | *MAT*α *thi20*Δ:: *NAT-STM#231* | H99 | 51297 / KCCM |
| 192 | YSB3220 | *MAT*α *thi20*Δ:: *NAT-STM#231* | H99 | 51297 / KCCM |
| 193 | YSB 1468 | *MAT*α thi6Δ:: *NAT-STM#290* | H99 | 51297 / KCCM |
| 194 | YSB 1469 | *MAT*α thi6Δ:: *NAT-STM#290* | H99 | 51297 / KCCM |
| 195 | YSB2443 | *MAT*α *tpk202a*Δ:: *NAT-STM#208* | H99 | 51297 / KCCM |
| 196 | YSB2444 | *MAT*α *tpk202a*Δ:: *NAT-STM#208* | H99 | 51297 / KCCM |
| 197 | YSB2892 | *MAT*α *utr1*Δ:: *NAT-STM#5* | H99 | 51297 / KCCM |
| 198 | YSB2893 | *MAT*α *utr1*Δ:: *NAT-STM#5* | H99 | 51297 / KCCM |
| 199 | YSB1500 | *MAT*α *vps15*Δ:: *NAT-STM#123* | H99 | 51297 / KCCM |
| 200 | YSB1501 | *MAT*α *vps15*Δ:: *NAT-STM#123* | H99 | 51297 / KCCM |
| 201 | YSB4180 | *MAT*α *xks1*Δ:: *NAT-STM#123* | H99 | 51297 / KCCM |
| 202 | YSB4181 | *MAT*α *xks1*Δ:: *NAT-STM#123* | H99 | 51297 / KCCM |
| 203 | YSB3736 | *MAT*α *yak103*Δ:: *NAT-STM#231* | H99 | 51297 / KCCM |
| 204 | YSB3737 | *MAT*α *yak103*Δ:: *NAT-STM#231* | H99 | 51297 / KCCM |
| 205 | YSB4275 | *MAT*α *yck2*Δ:: *NAT-STM#58* | H99 | 51297 / KCCM |
| 206 | YSB4278 | *MAT*α *yck2*Δ:: *NAT-STM#58* | H99 | 51297 / KCCM |
| 207 | YSB4332 | *MAT*α *yef1*Δ:: *NAT-STM#224* | H99 | 51297 / KCCM |
| 208 | YSB4333 | *MAT*α *yef1*Δ:: *NAT-STM#224* | H99 | 51297 / KCCM |
| 209 | YSB4294 | *MAT*α *yfh7*Δ:: *NAT-STM#295* | H99 | 51297 / KCCM |
| 210 | YSB4295 | *MAT*α *yfh7*Δ:: *NAT-STM#295* | H99 | 51297 / KCCM |
| 211 | YSB3926 | *MAT*α *ykl1*Δ:: *NAT-STM#122* | H99 | 51297 / KCCM |
| 212 | YSB3927 | *MAT*α *ykl1*Δ:: *NAT-STM#122* | H99 | 51297 / KCCM |
| 213 | YSB 1885 | *MAT*α *ypk101*Δ:: *NAT-STM#242* | H99 | 51297 / KCCM |
| 214 | YSB 1886 | *MAT*α *ypk101*Δ:: *NAT-STM#242* | H99 | 51297 / KCCM |
| 215 | YSB 1736 | *MAT*α *ypk1*Δ:: *NAT-STM#58* | H99 | 51297 / KCCM |
| 216 | YSB1737 | *MAT*α *ypk1*Δ:: *NAT-STM#58* | H99 | 51297 / KCCM |
| 217 | YSB 1429 | *MAT*α *apn2*Δ:: *NAT-STM#102* | H99 | 51291 / KCCM |
| 218 | YSB 1430 | *MAT*α *apn2*Δ:: *NAT-STM#102* | H99 | 51291 / KCCM |
| 219 | YSB714 | *MAT*α *aro80*Δ:: *NAT-STM#225* | H99 | 51291 / KCCM |
| 220 | YSB715 | *MAT*α *aro80*Δ:: *NAT-STM#225* | H99 | 51291 / KCCM |
| 221 | YSB661 | *MAT*α *aro8001*Δ:: *NAT-STM#225* | H99 | 51291 / KCCM |
| 222 | YSB662 | *MAT*α *aro8001*Δ:: *NAT-STM#225* | H99 | 51291 / KCCM |
| 223 | YSB3013 | *MAT*α *asg1*Δ:: *NAT-STM#6* | H99 | 51291 / KCCM |
| 224 | YSB3014 | *MAT*α *asg1*Δ:: *NAT-STM#6* | H99 | 51291 / KCCM |
| 225 | YSB2697 | *MAT*α *asg101*Δ:: *NAT-STM#150* | H99 | 51291 / KCCM |
| 226 | YSB2698 | *MAT*α *asg101*Δ:: *NAT-STM#150* | H99 | 51291 / KCCM |
| 227 | YSB 1839 | *MAT*α *bwc2*Δ:: *NAT-STM#184* | H99 | 51291 / KCCM |
| 228 | YSB 1840 | *MAT*α *bwc2*Δ:: *NAT-STM#184* | H99 | 51291 / KCCM |
| 229 | YSB723 | *MAT*α *bzp1*( *hxl1*)Δ:: *NAT-STM#295* | H99 | 51291 / KCCM |
| 230 | YSB724 | *MAT*α *bzp1*( *hxl1*)Δ:: *NAT-STM#295* | H99 | 51291 / KCCM |
| 231 | YSB2702 | *MAT*α *bzp2*Δ:: *NAT-STM#205* | H99 | 51291 / KCCM |
| 232 | YSB2703 | *MAT*α *bzp2*Δ:: *NAT-STM#205* | H99 | 51291 / KCCM |
| 233 | YSB 1099 | *MAT*α *bzp3*Δ:: *NAT-STM#146* | H99 | 51291 / KCCM |
| 234 | YSB1100 | *MAT*α *bzp3*Δ:: *NAT-STM#146* | H99 | 51291 / KCCM |
| 235 | YSB 1894 | *MAT*α *bzp4*Δ:: *NAT-STM#295* | H99 | 51291 / KCCM |
| 236 | YSB1895 | *MAT*α *bzp4*Δ:: *NAT-STM#295* | H99 | 51291 / KCCM |
| 237 | YSB 1474 | *MAT*α *bzp5*Δ:: *NAT-STM#191* | H99 | 51291 / KCCM |
| 238 | YSB 1475 | *MAT*α *bzp5*Δ:: *NAT-STM#191* | H99 | 51291 / KCCM |
| 239 | YSB706 | *MAT*α *ccd4*Δ:: *NAT-STM#122* | H99 | 51291 / KCCM |
| 240 | YSB707 | *MAT*α *ccd4*Δ:: *NAT-STM#122* | H99 | 51291 / KCCM |
| 241 | YSB847 | *MAT*α *cep3*Δ:: *NAT-STM#292* | H99 | 51291 / KCCM |
| 242 | YSB848 | *MAT*α *cep3*Δ:: *NAT-STM#292* | H99 | 51291 / KCCM |
| 243 | YSB 1396 | *MAT*α *clr1*Δ:: *NAT-STM#242* | H99 | 51291 / KCCM |
| 244 | YSB 1397 | *MAT*α *clr1*Δ:: *NAT-STM#242* | H99 | 51291 / KCCM |
| 245 | YSB 1834 | *MAT*α *clr3*Δ:: *NAT-STM#102* | H99 | 51291 / KCCM |
| 246 | YSB1836 | *MAT*α *clr3*Δ:: *NAT-STM#102* | H99 | 51291 / KCCM |
| 247 | YSB3282 | *MAT*α *clr4*Δ:: *NAT-STM#242* | H99 | 51291 / KCCM |
| 248 | YSB3283 | *MAT*α *clr4*Δ:: *NAT-STM#242* | H99 | 51291 / KCCM |
| 249 | YSB1106 | *MAT*α *clr6*Δ:: *NAT-STM#231* | H99 | 51291 / KCCM |
| 250 | YSB1107 | *MAT*α *clr6*Δ:: *NAT-STM#231* | H99 | 51291 / KCCM |
| 251 | YSB2665 | *MAT*α *cuf1*Δ:: *NAT-STM#205* | H99 | 51291 / KCCM |
| 252 | YSB2666 | *MAT*α *cuf1*Δ:: *NAT-STM#205* | H99 | 51291 / KCCM |
| 253 | YSB3150 | *MAT*α *ddt1*Δ:: *NAT-STM#102* | H99 | 51291 / KCCM |
| 254 | YSB3151 | *MAT*α *ddt1*Δ:: *NAT-STM#102* | H99 | 51291 / KCCM |
| 255 | YSB476 | *MAT*α *ecm22*Δ:: *NAT-STM#219* | H99 | 51291 / KCCM |
| 256 | YSB478 | *MAT*α *ecm22*Δ:: *NAT-STM#219* | H99 | 51291 / KCCM |
| 257 | YSB693 | *MAT*α *ert1*Δ:: *NAT-STM#225* | H99 | 51291 / KCCM |
| 258 | YSB694 | *MAT*α *ert1*Δ:: *NAT-STM#225* | H99 | 51291 / KCCM |
| 259 | YSB813 | *MAT*α *fap1*Δ:: *NAT-STM#296* | H99 | 51291 / KCCM |
| 260 | YSB817 | *MAT*α *fap1*Δ:: *NAT-STM#296* | H99 | 51291 / KCCM |
| 261 | YSB 1856 | *MAT*α *fkh101*Δ:: *NAT-STM#184* | H99 | 51291 / KCCM |
| 262 | YSB 1857 | *MAT*α *fkh101*Δ:: *NAT-STM#184* | H99 | 51291 / KCCM |
| 263 | YSB 1339 | *MAT*α *fkh2*Δ:: *NAT-STM#219* | H99 | 51291 / KCCM |
| 264 | YSB 1340 | *MAT*α *fkh2*Δ:: *NAT-STM#219* | H99 | 51291 / KCCM |
| 265 | YSB 1050 | *MAT*α *fzc2*Δ:: *NAT-STM#288* | H99 | 51291 / KCCM |
| 266 | YSB1051 | *MAT*α *fzc2*Δ:: *NAT-STM#288* | H99 | 51291 / KCCM |
| 267 | YSB2611 | *MAT*α *fzc3*Δ:: *NAT-STM#204* | H99 | 51291 / KCCM |
| 268 | YSB2664 | *MAT*α *fzc3*Δ:: *NAT-STM#204* | H99 | 51291 / KCCM |
| 269 | YSB2724 | *MAT*α *fzc4*Δ:: *NAT-STM#166* | H99 | 51291 / KCCM |
| 270 | YSB2725 | *MAT*α *fzc4*Δ:: *NAT-STM#166* | H99 | 51291 / KCCM |
| 271 | YSB 1400 | *MAT*α *fzc5*Δ:: *NAT-STM#5* | H99 | 51291 / KCCM |
| 272 | YSB 1401 | *MAT*α *fzc5*Δ:: *NAT-STM#5* | H99 | 51291 / KCCM |
| 273 | YSB 1980 | *MAT*α *fzc6*Δ:: *NAT-STM#211* | H99 | 51291 / KCCM |
| 274 | YSB 1981 | *MAT*α *fzc6*Δ:: *NAT-STM#211* | H99 | 51291 / KCCM |
| 275 | YSB2704 | *MAT*α *fzc7*Δ:: *NAT-STM#119* | H99 | 51291 / KCCM |
| 276 | YSB2705 | *MAT*α *fzc7*Δ:: *NAT-STM#119* | H99 | 51291 / KCCM |
| 277 | YSB2112 | *MAT*α *fzc8*Δ:: *NAT-STM#177* | H99 | 51291 / KCCM |
| 278 | YSB2113 | *MAT*α *fzc8*Δ:: *NAT-STM#177* | H99 | 51291 / KCCM |
| 279 | YSB3083 | *MAT*α *fzc101*Δ:: *NAT-STM#123* | H99 | 51291 / KCCM |
| 280 | YSB3368 | *MAT*α *fzc10*Δ:: *NAT-STM#123* | H99 | 51291 / KCCM |
| 281 | YSB845 | *MAT*α *fzc11*Δ:: *NAT-STM#292* | H99 | 51291 / KCCM |
| 282 | YSB846 | *MAT*α *fzc11*Δ:: *NAT-STM#292* | H99 | 51291 / KCCM |
| 283 | YSB467 | *MAT*α *fzc12*Δ:: *NAT-STM#224* | H99 | 51291 / KCCM |
| 284 | YSB468 | *MAT*α *fzc12*Δ:: *NAT-STM#224* | H99 | 51291 / KCCM |
| 285 | YSB2517 | *MAT*α *fzc13*Δ:: *NAT-STM#191* | H99 | 51291 / KCCM |
| 286 | YSB2518 | *MAT*α *fzc13*Δ:: *NAT-STM#191* | H99 | 51291 / KCCM |
| 287 | YSB 1846 | *MAT*α *fzc14*Δ:: *NAT-STM#43* | H99 | 51291 / KCCM |
| 288 | YSB 1847 | *MAT*α *fzc14*Δ:: *NAT-STM#43* | H99 | 51291 / KCCM |
| 289 | YSB646 | *MAT*α *fzc15*Δ:: *NAT-STM#122* | H99 | 51291 / KCCM |
| 290 | YSB647 | *MAT*α *fzc15*Δ:: *NAT-STM#122* | H99 | 51291 / KCCM |
| 291 | YSB2326 | *MAT*α *fzc16*Δ:: *NAT-STM#212* | H99 | 51291 / KCCM |
| 292 | YSB2327 | *MAT*α *fzc16*Δ:: *NAT-STM#212* | H99 | 51291 / KCCM |
| 293 | YSB2250 | *MAT*α *fzc17*Δ:: *NAT-STM#240* | H99 | 51291 / KCCM |
| 294 | YSB2251 | *MAT*α *fzc17*Δ:: *NAT-STM#240* | H99 | 51291 / KCCM |
| 295 | YSB2320 | *MAT*α *fzc18*Δ:: *NAT-STM#212* | H99 | 51291 / KCCM |
| 296 | YSB2321 | *MAT*α *fzc18*Δ:: *NAT-STM#212* | H99 | 51291 / KCCM |
| 297 | YSB2115 | *MAT*α *fzc19*Δ:: *NAT-STM#184* | H99 | 51291 / KCCM |
| 298 | YSB2116 | *MAT*α *fzc19*Δ:: *NAT-STM#184* | H99 | 51291 / KCCM |
| 299 | YSB3128 | *MAT*α*fzc20*Δ:: *NAT-STM#191* | H99 | 51291 / KCCM |
| 300 | YSB3129 | *MAT*α*fzc20*Δ:: *NAT-STM#191* | H99 | 51291 / KCCM |
| 301 | YSB 1252 | *MAT*α*fzc21*Δ:: *NAT-STM#150* | H99 | 51291 / KCCM |
| 302 | YSB 1253 | *MAT*α*fzc21*Δ:: *NAT-STM#150* | H99 | 51291 / KCCM |
| 303 | YSB 1688 | *MAT*α*fzc22*Δ:: *NAT-STM#273* | H99 | 51291 / KCCM |
| 304 | YSB2974 | *MAT*α*fzc22*Δ:: *NAT-STM#273* | H99 | 51291 / KCCM |
| 305 | YSB3105 | *MAT*α*fzc23*Δ:: *NAT-STM#201* | H99 | 51291 / KCCM |
| 306 | YSB3106 | *MAT*α*fzc23*Δ:: *NAT-STM#201* | H99 | 51291 / KCCM |
| 307 | YSB774 | *MAT*α*fzc24*Δ:: *NAT-STM#292* | H99 | 51291 / KCCM |
| 308 | YSB775 | *MAT*α*fzc24*Δ:: *NAT-STM#292* | H99 | 51291 / KCCM |
| 309 | YSB518 | *MAT*α*fzc25*Δ:: *NAT-STM#227* | H99 | 51291 / KCCM |
| 310 | YSB1822 | *MAT*α*fzc25*Δ:: *NAT-STM#227* | H99 | 51291 / KCCM |
| 311 | YSB3084 | *MAT*α*fzc26*Δ:: *NAT-STM#146* | H99 | 51291 / KCCM |
| 312 | YSB3085 | *MAT*α*fzc26*Δ:: *NAT-STM#146* | H99 | 51291 / KCCM |
| 313 | YSB582 | *MAT*α*fzc27*Δ:: *NAT-STM#220* | H99 | 51291 / KCCM |
| 314 | YSB583 | *MAT*α*fzc27*Δ:: *NAT-STM#220* | H99 | 51291 / KCCM |
| 315 | YSB2337 | *MAT*α*fzc28*Δ:: *NAT-STM#125* | H99 | 51291 / KCCM |
| 316 | YSB2338 | *MAT*α*fzc28*Δ:: *NAT-STM#125* | H99 | 51291 / KCCM |
| 317 | YSB718 | *MAT*α*fzc29*Δ:: *NAT-STM#225* | H99 | 51291 / KCCM |
| 318 | YSB719 | *MAT*α*fzc29*Δ:: *NAT-STM#225* | H99 | 51291 / KCCM |
| 319 | YSB2447 | *MAT*α*fzc30*Δ:: *NAT-STM#230* | H99 | 51291 / KCCM |
| 320 | YSB2448 | *MAT*α*fzc30*Δ:: *NAT-STM#230* | H99 | 51291 / KCCM |
| 321 | YSB2385 | *MAT*α*fzc32*Δ:: *NAT-STM#234* | H99 | 51291 / KCCM |
| 322 | YSB2526 | *MAT*α*fzc32*Δ:: *NAT-STM#234* | H99 | 51291 / KCCM |
| 323 | YSB 1074 | *MAT*α*fzc33*Δ:: *NAT-STM#43* | H99 | 51291 / KCCM |
| 324 | YSB1075 | *MAT*α*fzc33*Δ:: *NAT-STM#43* | H99 | 51291 / KCCM |
| 325 | YSB501 | *MAT*α*fzc34*Δ:: *NAT-STM#231* | H99 | 51291 / KCCM |
| 326 | YSB2979 | *MAT*α*fzc34*Δ:: *NAT-STM#231* | H99 | 51291 / KCCM |
| 327 | YSB 1341 | *MAT*α*fzc35*Δ:: *NAT-STM#213* | H99 | 51291 / KCCM |
| 328 | YSB 1342 | *MAT*α*fzc35*Δ:: *NAT-STM#213* | H99 | 51291 / KCCM |
| 329 | YSB2335 | *MAT*α*fzc36*Δ:: *NAT-STM#119* | H99 | 51291 / KCCM |
| 330 | YSB2523 | *MAT*α*fzc36*Δ:: *NAT-STM#119* | H99 | 51291 / KCCM |
| 331 | YSB 1329 | *MAT*α*fzc37*Δ:: *NAT-STM#210* | H99 | 51291 / KCCM |
| 332 | YSB 1330 | *MAT*α*fzc37*Δ:: *NAT-STM#210* | H99 | 51291 / KCCM |
| 333 | YSB777 | *MAT*α*fzc38*Δ:: *NAT-STM#292* | H99 | 51291 / KCCM |
| 334 | YSB 1330 | *MAT*α*fzc38*Δ:: *NAT-STM#292* | H99 | 51291 / KCCM |
| 335 | YSB 1820 | *MAT*α*fzc39*Δ:: *NAT-STM#231* | H99 | 51291 / KCCM |
| 336 | YSB2621 | *MAT*α*fzc39*Δ:: *NAT-STM#231* | H99 | 51291 / KCCM |
| 337 | YSB3088 | *MAT*α*fzc40*Δ:: *NAT-STM#205* | H99 | 51291 / KCCM |
| 338 | YSB3758 | *MAT*α*fzc40*Δ:: *NAT-STM#205* | H99 | 51291 / KCCM |
| 339 | YSB1334 | *MAT*α*fzc41*Δ:: *NAT-STM#295* | H99 | 51291 / KCCM |
| 340 | YSB1335 | *MAT*α*fzc41*Δ:: *NAT-STM#295* | H99 | 51291 / KCCM |
| 341 | YSB687 | *MAT*α*fzc42*Δ:: *NAT-STM#122* | H99 | 51291 / KCCM |
| 342 | YSB690 | *MAT*α*fzc42*Δ:: *NAT-STM#122* | H99 | 51291 / KCCM |
| 343 | YSB517 | *MAT*α*fzc43*Δ:: *NAT-STM#191* | H99 | 51291 / KCCM |
| 344 | YSB2334 | *MAT*α*fzc43*Δ:: *NAT-STM#191* | H99 | 51291 / KCCM |
| 345 | YSB2182 | *MAT*α*fzc44*Δ:: *NAT-STM#5* | H99 | 51291 / KCCM |
| 346 | YSB2183 | *MAT*α*fzc44*Δ:: *NAT-STM#5* | H99 | 51291 / KCCM |
| 347 | YSB2221 | *MAT*α*fzc45*Δ:: *NAT-STM#58* | H99 | 51291 / KCCM |
| 348 | YSB2222 | *MAT*α*fzc45*Δ:: *NAT-STM#58* | H99 | 51291 / KCCM |
| 349 | YSB1209 | *MAT*α*fzc46*Δ:: *NAT-STM#177* | H99 | 51291 / KCCM |
| 350 | YSB1210 | *MAT*α*fzc46*Δ:: *NAT-STM#177* | H99 | 51291 / KCCM |
| 351 | YSB 1406 | *MAT*α*fzc47*Δ:: *NAT-STM#102* | H99 | 51291 / KCCM |
| 352 | YSB 1407 | *MAT*α*fzc47*Δ:: *NAT-STM#102* | H99 | 51291 / KCCM |
| 353 | YSB2646 | *MAT*α*fzc48*Δ:: *NAT-STM#290* | H99 | 51291 / KCCM |
| 354 | YSB2647 | *MAT*α*fzc48*Δ:: *NAT-STM#290* | H99 | 51291 / KCCM |
| 355 | YSB2171 | *MAT*α*fzc49*Δ:: *NAT-STM#5* | H99 | 51291 / KCCM |
| 356 | YSB2173 | *MAT*α*fzc49*Δ:: *NAT-STM#5* | H99 | 51291 / KCCM |
| 357 | YSB3131 | *MAT*α*fzc50*Δ:: *NAT-STM#204* | H99 | 51291 / KCCM |
| 358 | YSB3132 | *MAT*α*fzc50*Δ:: *NAT-STM#204* | H99 | 51291 / KCCM |
| 359 | YSB1842 | *MAT*α*fzc51*Δ:: *NAT-STM#159* | H99 | 51291 / KCCM |
| 360 | YSB1843 | *MAT*α*fzc51*Δ:: *NAT-STM#159* | H99 | 51291 / KCCM |
| 361 | YSB2972 | *MAT*α*gat1*Δ:: *NAT-STM#227* | H99 | 51291 / KCCM |
| 362 | YSB2973 | *MAT*α*gat1*Δ:: *NAT-STM#227* | H99 | 51291 / KCCM |
| 363 | YSB569 | *MAT*α*gat203*Δ:: *NAT-STM#220* | H99 | 51291 / KCCM |
| 364 | YSB570 | *MAT*α*gat203*Δ:: *NAT-STM#220* | H99 | 51291 / KCCM |
| 365 | YSB 1311 | *MAT*α*gat204*Δ:: *NAT-STM#218* | H99 | 51291 / KCCM |
| 366 | YSB1312 | *MAT*α*gat204*Δ:: *NAT-STM#218* | H99 | 51291 / KCCM |
| 367 | YSB3033 | *MAT*α*gat5*Δ:: *NAT-STM#290* | H99 | 51291 / KCCM |
| 368 | YSB3034 | *MAT*α*gat5*Δ:: *NAT-STM#290* | H99 | 51291 / KCCM |
| 369 | YSB1385 | *MAT*α*gat6*Δ:: *NAT-STM#201* | H99 | 51291 / KCCM |
| 370 | YSB1386 | *MAT*α*gat6*Δ:: *NAT-STM#201* | H99 | 51291 / KCCM |
| 371 | YSB2699 | *MAT*α*gat7*Δ:: *NAT-STM#159* | H99 | 51291 / KCCM |
| 372 | YSB2700 | *MAT*α*gat7*Δ:: *NAT-STM#159* | H99 | 51291 / KCCM |
| 373 | YSB471 | *MAT*α*gat8*Δ:: *NAT-STM#125* | H99 | 51291 / KCCM |
| 374 | YSB472 | *MAT*α*gat8*Δ:: *NAT-STM#125* | H99 | 51291 / KCCM |
| 375 | YSB3154 | *MAT*α*gln3*Δ:: *NAT-STM#230* | H99 | 51291 / KCCM |
| 376 | YSB3155 | *MAT*α*gln3*Δ:: *NAT-STM#230* | H99 | 51291 / KCCM |
| 377 | YSB796 | *MAT*α*grf1*Δ:: *NAT-STM#296* | H99 | 51291 / KCCM |
| 378 | YSB797 | *MAT*α*grf1*Δ:: *NAT-STM#296* | H99 | 51291 / KCCM |
| 379 | YSB2481 | *MAT*α*hap1*Δ:: *NAT-STM#240* | H99 | 51291 / KCCM |
| 380 | YSB2482 | *MAT*α*hap1*Δ:: *NAT-STM#240* | H99 | 51291 / KCCM |
| 381 | YSB 1850 | *MAT*α*hcm1*Δ:: *NAT-STM#177* | H99 | 51291 / KCCM |
| 382 | YSB 1851 | *MAT*α*hcm1*Δ:: *NAT-STM#177* | H99 | 51291 / KCCM |
| 383 | YSB2390 | *MAT*α*hcm101*Δ:: *NAT-STM#211* | H99 | 51291 / KCCM |
| 384 | YSB2391 | *MAT*α*hcm101*Δ:: *NAT-STM#211* | H99 | 51291 / KCCM |
| 385 | YSB 1382 | *MAT*α*hel2*Δ:: *NAT-STM#204* | H99 | 51291 / KCCM |
| 386 | YSB1383 | *MAT*α*hel2*Δ:: *NAT-STM#204* | H99 | 51291 / KCCM |
| 387 | YSB 1175 | *MAT*α*hlh1*Δ:: *NAT-STM#146* | H99 | 51291 / KCCM |
| 388 | YSB1176 | *MAT*α*hlh1*Δ:: *NAT-STM#146* | H99 | 51291 / KCCM |
| 389 | YSB1147 | *MAT*α*hlh2*Δ:: *NAT-STM#224* | H99 | 51291 / KCCM |
| 390 | YSB1149 | *MAT*α*hlh2*Δ:: *NAT-STM#224* | H99 | 51291 / KCCM |
| 391 | YSB2244 | *MAT*α*hlh4*Δ:: *NAT-STM#295* | H99 | 51291 / KCCM |
| 392 | YSB2245 | *MAT*α*hlh4*Δ:: *NAT-STM#295* | H99 | 51291 / KCCM |
| 393 | YSB2609 | *MAT*α*hlhS*Δ:: *NAT-STM#210* | H99 | 51291 / KCCM |
| 394 | YSB3059 | *MAT*α*hlh5*Δ:: *NAT-STM#210* | H99 | 51291 / KCCM |
| 395 | YSB2282 | *MAT*α*hob2*Δ:: *NAT-STM#43* | H99 | 51291 / KCCM |
| 396 | YSB2283 | *MAT*α*hob2*Δ:: *NAT-STM#43* | H99 | 51291 / KCCM |
| 397 | YSB2001 | *MAT*α*hob3*Δ:: *NAT-STM#211* | H99 | 51291 / KCCM |
| 398 | YSB2002 | *MAT*α*hob3α*Δ:: *NAT-STM#211* | H99 | 51291 / KCCM |
| 399 | YSB1435 | *MAT*α*hob4*Δ:: *NAT-STM#159* | H99 | 51291 / KCCM |
| 400 | YSB1437 | *MAT*α*hob4*Δ:: *NAT-STM#159* | H99 | 51291 / KCCM |
| 401 | YSB 1255 | *MAT*α*hob6*Δ:: *NAT-STM#201* | H99 | 51291 / KCCM |
| 402 | YSB 1256 | *MAT*α*hob6*Δ:: *NAT-STM#201* | H99 | 51291 / KCCM |
| 403 | YSB3026 | *MAT*α*hob7*Δ:: *NAT-STM#159* | H99 | 51291 / KCCM |
| 404 | YSB3027 | *MAT*α*hob7*Δ:: *NAT-STM#159* | H99 | 51291 / KCCM |
| 405 | YSB2295 | *MAT*α*hsf2*Δ:: *NAT-STM#205* | H99 | 51291 / KCCM |
| 406 | YSB2296 | *MAT*α*hsf2*Δ:: *NAT-STM#205* | H99 | 51291 / KCCM |
| 407 | YSB2527 | *MAT*α*hsf3*Δ:: *NAT-STM#273* | H99 | 51291 / KCCM |
| 408 | YSB2528 | *MAT*α*hsf3*Δ:: *NAT-STM#273* | H99 | 51291 / KCCM |
| 409 | YSB2211 | *MAT*α*liv1*Δ:: *NAT-STM#213* | H99 | 51291 / KCCM |
| 410 | YSB2212 | *MAT*α*liv1*Δ:: *NAT-STM#213* | H99 | 51291 / KCCM |
| 411 | YSB2089 | *MAT*α*liv4*Δ:: *NAT-STM#234* | H99 | 51291 / KCCM |
| 412 | YSB2634 | *MAT*α*liv4*Δ:: *NAT-STM#234* | H99 | 51291 / KCCM |
| 413 | YSB506 | *MAT*α*mal13*Δ:: *NAT-STM#230* | H99 | 51291 / KCCM |
| 414 | YSB507 | *MAT*α*mal13*Δ:: *NAT-STM#230* | H99 | 51291 / KCCM |
| 415 | YSB768 | *MAT*α*mbf1*Δ:: *NAT-STM#296* | H99 | 51291 / KCCM |
| 416 | YSB769 | *MAT*α*mbf1*Δ:: *NAT-STM#296* | H99 | 51291 / KCCM |
| 417 | YSB488 | *MAT*α*mbs1*Δ:: *NAT-STM#150* | H99 | 51291 / KCCM |
| 418 | YSB489 | *MAT*α*mbs1*Δ:: *NAT-STM#150* | H99 | 51291 / KCCM |
| 419 | YSB538 | *MAT*α*mbs2*Δ:: *NAT-STM#122* | H99 | 51291 / KCCM |
| 420 | YSB539 | *MAT*α*mbs2*Δ:: *NAT-STM#122* | H99 | 51291 / KCCM |
| 421 | YSB 1302 | *MAT*α*mcm1*Δ:: *NAT-STM#218* | H99 | 51291 / KCCM |
| 422 | YSB 1303 | *MAT*α*mcm1*Δ:: *NAT-STM#218* | H99 | 51291 / KCCM |
| 423 | YSB 1178 | *MAT*α*met32*Δ:: *NAT-STM#58* | H99 | 51291 / KCCM |
| 424 | YSB1179 | *MAT*α*met32*Δ:: *NAT-STM#58* | H99 | 51291 / KCCM |
| 425 | YSB2133 | *MAT*α*miz1*Δ:: *NAT-STM#210* | H99 | 51291 / KCCM |
| 426 | YSB3366 | *MAT*α*miz1*Δ:: *NAT-STM#210* | H99 | 51291 / KCCM |
| 427 | YSB 1172 | *MAT*α*mln1*Δ:: *NAT-STM#146* | H99 | 51291 / KCCM |
| 428 | YSB 1173 | *MAT*α*mln1*Δ:: *NAT-STM#146* | H99 | 51291 / KCCM |
| 429 | YSB2727 | *MAT*α*mlr1*Δ:: *NAT-STM#116* | H99 | 51291 / KCCM |
| 430 | YSB2728 | *MAT*α*mlr1*Δ:: *NAT-STM#116* | H99 | 51291 / KCCM |
| 431 | YSB3096 | *MAT*α*nrg1*Δ:: *NAT-STM#123* | H99 | 51291 / KCCM |
| 432 | YSB3097 | *MAT*α*nrg1*Δ:: *NAT-STM#123* | H99 | 51291 / KCCM |
| 433 | YSB 1181 | *MAT*α*pan1*Δ:: *NAT-STM#242* | H99 | 51291 / KCCM |
| 434 | YSB 1183 | *MAT*α*pan1*Δ:: *NAT-STM#242* | H99 | 51291 / KCCM |
| 435 | YSB 1249 | *MAT*α*pip2*Δ:: *NAT-STM#232* | H99 | 51291 / KCCM |
| 436 | YSB1250 | *MAT*α*pip2*Δ:: *NAT-STM#232* | H99 | 51291 / KCCM |
| 437 | YSB3099 | *MAT*α*pip201*Δ:: *NAT-STM#123* | H99 | 51291 / KCCM |
| 438 | YSB3100 | *MAT*α*pip201*Δ:: *NAT-STM#123* | H99 | 51291 / KCCM |
| 439 | YSB1046 | *MAT*α*ppr1*Δ:: *NAT-STM#288* | H99 | 51291 / KCCM |
| 440 | YSB1047 | *MAT*α*ppr1*Δ:: *NAT-STM#288* | H99 | 51291 / KCCM |
| 441 | YSB 1898 | *MAT*α*rds2*Δ:: *NAT-STM#242* | H99 | 51291 / KCCM |
| 442 | YSB 1899 | *MAT*α*rds2*Δ:: *NAT-STM#242* | H99 | 51291 / KCCM |
| 443 | YSB 1366 | *MAT*α*rim101*Δ:: *NAT-STM#208* | H99 | 51291 / KCCM |
| 444 | YSB 1367 | *MAT*α*rim101*Δ:: *NAT-STM#208* | H99 | 51291 / KCCM |
| 445 | YSB 1300 | *MAT*α*rlm1*Δ:: *NAT-STM#234* | H99 | 51291 / KCCM |
| 446 | YSB1301 | *MAT*α*rlm1*Δ:: *NAT-STM#234* | H99 | 51291 / KCCM |
| 447 | YSB3164 | *MAT*α*rum1*Δ:: *NAT-STM#288* | H99 | 51291 / KCCM |
| 448 | YSB3747 | *MAT*α*rum1*Δ:: *NAT-STM#288* | H99 | 51291 / KCCM |
| 449 | YSB2680 | *MAT*α*sip4*Δ:: *NAT-STM#290* | H99 | 51291 / KCCM |
| 450 | YSB2681 | *MAT*α*sip4*Δ:: *NAT-STM#290* | H99 | 51291 / KCCM |
| 451 | YSB 1358 | *MAT*α*sip401*Δ:: *NAT-STM#58* | H99 | 51291 / KCCM |
| 452 | YSB 1359 | *MAT*α*sip401*Δ:: *NAT-STM#58* | H99 | 51291 / KCCM |
| 453 | YSB529 | *MAT*α*sip402*Δ:: *NAT-STM#270* | H99 | 51291 / KCCM |
| 454 | YSB530 | *MAT*α*sip402*Δ:: *NAT-STM#270* | H99 | 51291 / KCCM |
| 455 | YSB349 | *MAT*α*skn7*Δ:: *NAT-STM#201* | H99 | 51291 / KCCM |
| 456 | YSB350 | *MAT*α*skn7*Δ:: *NAT-STM#201* | H99 | 51291 / KCCM |
| 457 | YSB 1542 | *MAT*α*ste12*Δ:: *NAT-STM#58* | H99 | 51291 / KCCM |
| 458 | YSB 1543 | *MAT*α*ste12*Δ:: *NAT-STM#58* | H99 | 51291 / KCCM |
| 459 | YSB1390 | *MAT*α*sxl1alpha*Δ:: *NAT-STM#208* | H99 | 51291 / KCCM |
| 460 | YSB1391 | *MAT*α*sxl1alpha*Δ:: *NAT-STM#208* | H99 | 51291 / KCCM |
| 461 | YSB 1464 | *MAT*α*usv101*Δ:: *NAT-STM#191* | H99 | 51291 / KCCM |
| 462 | YSB 1465 | *MAT*α*usv101*Δ:: *NAT-STM#191* | H99 | 51291 / KCCM |
| 463 | YSB815 | *MAT*α*yap1*Δ:: *NAT-STM#296* | H99 | 51291 / KCCM |
| 464 | YSB 1290 | *MAT*α*yap1*Δ:: *NAT-STM#296* | H99 | 51291 / KCCM |
| 465 | YSB 1416 | *MAT*α*yap2*Δ:: *NAT-STM#218* | H99 | 51291 / KCCM |
| 466 | YSB1417 | *MAT*α*yap2*Δ:: *NAT-STM#218* | H99 | 51291 / KCCM |
| 467 | YSB3134 | *MAT*α*yox101*Δ:: *NAT-STM#227* | H99 | 51291 / KCCM |
| 468 | YSB3136 | *MAT*α*yox101*Δ:: *NAT-STM#227* | H99 | 51291 / KCCM |
| 469 | YSB2997 | *MAT*α*yrm101*Δ:: *NAT-STM#219* | H99 | 51291 / KCCM |
| 470 | YSB2998 | *MAT*α*yrm101*Δ:: *NAT-STM#219* | H99 | 51291 / KCCM |
| 471 | YSB2298 | *MAT*α*yrm103*Δ:: *NAT-STM#5* | H99 | 51291 / KCCM |
| 472 | YSB2299 | *MAT*α*yrm103*Δ:: *NAT-STM#5* | H99 | 51291 / KCCM |
| 473 | YSB2540 | *MAT*α*zap103*Δ:: *NAT-STM#234* | H99 | 51291 / KCCM |
| 474 | YSB2541 | *MAT*α*zap103*Δ:: *NAT-STM#234* | H99 | 51291 / KCCM |
| 475 | YSB2134 | *MAT*α*zap104*Δ:: *NAT-STM#204* | H99 | 51291 / KCCM |
| 476 | YSB2135 | *MAT*α*zap104*Δ:: *NAT-STM#204* | H99 | 51291 / KCCM |
| 477 | YSB2573 | *MAT*α*zfc1*Δ:: *NAT-STM#224* | H99 | 51291 / KCCM |
| 478 | YSB2574 | *MAT*α*zfc1*Δ:: *NAT-STM#224* | H99 | 51291 / KCCM |
| 479 | YSB2622 | *MAT*α*zfc2*Δ:: *NAT-STM#6* | H99 | 51291 / KCCM |
| 480 | YSB2623 | *MAT*α*zfc2*Δ:: *NAT-STM#6* | H99 | 51291 / KCCM |
| 481 | YSB2231 | *MAT*α*zfc4*Δ:: *NAT-STM#210* | H99 | 51291 / KCCM |
| 482 | YSB2232 | *MAT*α*zfc4*Δ:: *NAT-STM#210* | H99 | 51291 / KCCM |
| 483 | YSB2177 | *MAT*α*zfc5*Δ:: *NAT-STM#6* | H99 | 51291 / KCCM |
| 484 | YSB2178 | *MAT*α*zfc5*Δ:: *NAT-STM#6* | H99 | 51291 / KCCM |
| 485 | YSB 1953 | *MAT*α*zfc6*Δ:: *NAT-STM#177* | H99 | 51291 / KCCM |
| 486 | YSB1954 | *MAT*α*zfc6*Δ:: *NAT-STM#177* | H99 | 51291 / KCCM |
| 487 | YSB481 | *MAT*α*zfc7*Δ:: *NAT-STM#224* | H99 | 51291 / KCCM |
| 488 | YSB482 | *MAT*α*zfc7*Δ:: *NAT-STM#224* | H99 | 51291 / KCCM |
| 489 | YSB3031 | *MAT*α*zfc8*Δ:: *NAT-STM#230* | H99 | 51291 / KCCM |
| 490 | YSB3032 | *MAT*α*zfc8*Δ:: *NAT-STM#230* | H99 | 51291 / KCCM |
| 491 | YSB2740 | *MAT*α*znf2*Δ:: *NAT-STM#211* | H99 | 51291 / KCCM |
| 492 | YSB2741 | *MAT*α*znf2*Δ:: *NAT-STM#211* | H99 | 51291 / KCCM |
| 493 | YSB4327 | *MAT*α*cex1*Δ:: *NAT-STM#219* | H99 | 51291 / KCCM |
| 494 | YSB4328 | *MAT*α*cex1*Δ:: *NAT-STM#219* | H99 | 51291 / KCCM |
| 495 | YSB 1571 | *MAT*α*alk1*Δ:: *NAT-STM#122* | H99 | 51291 / KCCM |
| 496 | YSB1573 | *MAT*α*alk1*Δ:: *NAT-STM#122* | H99 | 51291 / KCCM |
| 497 | YSB 123 | *MAT*α*pbs2*Δ:: *NAT-STM#213* | H99 | 51291 / KCCM |
| 498 | YSB 124 | *MAT*α*pbs2*Δ:: *NAT-STM#213* | H99 | 51291 / KCCM |
| 499 | YSB2826 | *MAT*α*yfh701*Δ:: *NAT-STM#220* | H99 | 51291 / KCCM |
| 500 | YSB3716 | *MAT*α*yfh701*Δ:: *NAT-STM#220* | H99 | 51291 / KCCM |
| 501 | YSB 1234 | *MAT*α*pkh201*Δ:: *NAT-STM#219* | H99 | 51291 / KCCM |
| 502 | YSB 1235 | *MAT*α*pkh201*Δ:: *NAT-STM#219* | H99 | 51291 / KCCM |
| 503 | YSB2072 | *MAT*α*abc1*Δ:: *NAT-STM#119* | H99 | 51291 / KCCM |
| 504 | YSB2797 | *MAT*α*abc1*Δ:: *NAT-STM#119* | H99 | 51291 / KCCM |
| 505 | YSB3056 | *MAT*α*trm7*Δ:: *NAT-STM#102* | H99 | 51291 / KCCM |
| 506 | YSB3057 | *MAT*α*trm7*Δ:: *NAT-STM#102* | H99 | 51291 / KCCM |
| 507 | YSB3153 | *MAT*α*tlk1*Δ:: *NAT-STM#116* | H99 | 51291 / KCCM |
| 508 | YSB3188 | *MAT*α*tlk1*Δ:: *NAT-STM#116* | H99 | 51291 / KCCM |
| 509 | YSB3824 | *MAT*α*mak3201*Δ:: *NAT-STM#159* | H99 | 51291 / KCCM |
| 510 | YSB3825 | *MAT*α*mak3201*Δ:: *NAT-STM#159* | H99 | 51291 / KCCM |
| 511 | YSB 1709 | *MAT*α*crk1*Δ:: *NAT-STM#43* | H99 | 51291 / KCCM |
| 512 | YSB1710 | *MAT*α*crk1*Δ:: *NAT-STM#43* | H99 | 51291 / KCCM |
| 513 | YSB3329 | *MAT*α*met3*Δ:: *NAT-STM#205* | H99 | 51291 / KCCM |
| 514 | YSB3330 | *MAT*α*met3*Δ:: *NAT-STM#205* | H99 | 51291 / KCCM |
| 515 | YSB 1800 | *MAT*α*hsl101*Δ:: *NAT-STM#146* | H99 | 51291 / KCCM |
| 516 | YSB1801 | *MAT*α*hsl101*Δ:: *NAT-STM#146* | H99 | 51291 / KCCM |
| 517 | YSB2372 | *MAT*α*snf1*Δ:: *NAT-STM#204* | H99 | 51291 / KCCM |
| 518 | YSB2373 | *MAT*α*snf1*Δ:: *NAT-STM#204* | H99 | 51291 / KCCM |
| 519 | YSB619 | *MAT*α*sch9*Δ:: *NAT-STM#169* | H99 | 51291 / KCCM |
| 520 | YSB620 | *MAT*α*sch9*Δ:: *NAT-STM#169* | H99 | 51291 / KCCM |
| 521 | YSB2952 | *MAT*α*irk5*Δ:: *NAT-STM#213* | H99 | 51291 / KCCM |
| 522 | YSB2953 | *MAT*α*irk5*Δ:: *NAT-STM#213* | H99 | 51291 / KCCM |
| 523 | YSB2216 | *MAT*α*vrk1*Δ:: *NAT-STM#123* | H99 | 51291 / KCCM |
| 524 | YSB2217 | *MAT*α*vrk1*Δ:: *NAT-STM#123* | H99 | 51291 / KCCM |
| 525 | YSB2415 | *MAT*α*gal83*Δ:: *NAT-STM#288* | H99 | 51291 / KCCM |
| 526 | YSB2416 | *MAT*α*gal83*Δ:: *NAT-STM#288* | H99 | 51291 / KCCM |
| 527 | YSB 1266 | *MAT*α*urk1*Δ:: *NAT-STM#43* | H99 | 51291 / KCCM |
| 528 | YSB 1267 | *MAT*α*urk1*Δ:: *NAT-STM#43* | H99 | 51291 / KCCM |
| 529 | YSB1904 | *MAT*α*irk2*Δ:: *NAT-STM#232* | H99 | 51291 / KCCM |
| 530 | YSB1905 | *MAT*α*irk2*Δ:: *NAT-STM#232* | H99 | 51291 / KCCM |
| 531 | YSB2040 | *MAT*α*yak1*Δ:: *NAT-STM#184* | H99 | 51291 / KCCM |
| 532 | YSB2096 | *MAT*α*yak1*Δ:: *NAT-STM#184* | H99 | 51291 / KCCM |
| 533 | YSB64 | *MAT*α*hog1*Δ:: *NAT-STM#177* | H99 | 51291 / KCCM |
| 534 | YSB65 | *MAT*α*hog1*Δ:: *NAT-STM#177* | H99 | 51291 / KCCM |
| 535 | YSB264 | *MAT*α*ssk2*Δ:: *NAT-STM#210* | H99 | 51291 / KCCM |
| 536 | YSB265 | *MAT*α*ssk2*Δ:: *NAT-STM#210* | H99 | 51291 / KCCM |
| 537 | YSB 1912 | *MAT*α*dak101*Δ:: *NAT-STM#282* | H99 | 51291 / KCCM |
| 538 | YSB1913 | *MAT*α*dak101*Δ:: *NAT-STM#282* | H99 | 51291 / KCCM |
| 539 | YSB3930 | *MAT*α*kin1*Δ:: *NAT-STM#6* | H99 | 51291 / KCCM |
| 540 | YSB3931 | *MAT*α*kin1*Δ:: *NAT-STM#6* | H99 | 51291 / KCCM |
| 541 | YSB188 | *MAT*α*pka1*Δ:: *NAT-STM#191* | H99 | 51291 / KCCM |
| 542 | YSB189 | *MAT*α*pka1*Δ:: *NAT-STM#191* | H99 | 51291 / KCCM |
| 543 | YSB 1104 | *MAT*α*hap2*Δ:: *NAT-STM#123* | H99 | 51291 / KCCM |
| 544 | YSB 1105 | *MAT*α*hap2*Δ:: *NAT-STM#123* | H99 | 51291 / KCCM |
| 545 | YSB2381 | *MAT*α*ada2*Δ:: *NAT-STM#232* | H99 | 51291 / KCCM |
| 546 | YSB2382 | *MAT*α*ada2*Δ:: *NAT-STM#232* | H99 | 51291 / KCCM |
| 547 | YSB1592 | *MAT*α*jjj1*Δ:: *NAT-STM#240* | H99 | 51291 / KCCM |
| 548 | YSB1594 | *MAT*α*jjj1*Δ:: *NAT-STM#240* | H99 | 51291 / KCCM |
| 549 | YSB2329 | *MAT*α*pho4*/*hlh3*Δ:: *NAT-STM#208* | H99 | 51291 / KCCM |
| 550 | YSB2330 | *MAT*α*pho4*/*hlh3*Δ:: *NAT-STM#208* | H99 | 51291 / KCCM |
| 551 | YSB676 | *MAT*α*atf1*Δ:: *NAT-STM#220* | H99 | 51291 / KCCM |
| 552 | YSB678 | *MAT*α*atf1*Δ:: *NAT-STM#220* | H99 | 51291 / KCCM |
| 553 | YSB 1585 | *MAT*α*hob5*Δ:: *NAT-STM#219* | H99 | 51291 / KCCM |
| 554 | YSB 1586 | *MAT*α*hob*5Δ:: *NAT-STM#219* | H99 | 51291 / KCCM |
| 555 | YSB1013 | *MAT*α*stb4*Δ:: *NAT-STM#125* | H99 | 51291 / KCCM |
| 556 | YSB1014 | *MAT*α*stb4*Δ:: *NAT-STM#125* | H99 | 51291 / KCCM |
| 557 | YSB 1263 | *MAT*α*sp1(crz1)*Δ:: *NAT-STM#231* | H99 | 51291 / KCCM |
| 558 | YSB 1264 | *MAT*α*sp1(crz1)*Δ:: *NAT-STM#231* | H99 | 51291 / KCCM |
| 559 | YSB2108 | *MAT*α*zfc3*Δ:: *NAT-STM#232* | H99 | 51291 / KCCM |
| 560 | YSB2386 | *MAT*α*zfc3*Δ:: *NAT-STM#232* | H99 | 51291 / KCCM |
| 561 | YSB2494 | *MAT*α*sre1*Δ:: *NAT-STM#242* | H99 | 51291 / KCCM |
| 562 | YSB2493 | *MAT*α*sre1*Δ:: *NAT-STM#242* | H99 | 51291 / KCCM |
| 563 | YSB2308 | *MAT*α*hob1*Δ:: *NAT-STM#213* | H99 | 51291 / KCCM |
| 564 | YSB2309 | *MAT*α*hob1*Δ:: *NAT-STM#213* | H99 | 51291 / KCCM |
| 565 | YSB2387 | *MAT*α*pdr802*Δ:: *NAT-STM#220* | H99 | 51291 / KCCM |
| 566 | YSB2388 | *MAT*α*pdr802*Δ:: *NAT-STM#220* | H99 | 51291 / KCCM |
| 567 | YSB2984 | *MAT*α*fzc9*Δ:: *NAT-STM#232* | H99 | 51291 / KCCM |
| 568 | YSB3266 | *MAT*α*fzc9*Δ:: *NAT-STM#232* | H99 | 51291 / KCCM |
| 569 | YSB510 | *MAT*α*fzc1*Δ:: *NAT-STM#116* | H99 | 51291 / KCCM |
| 570 | YSB511 | *MAT*α*fzc1*Δ:: *NAT-STM#116* | H99 | 51291 / KCCM |
| 571 | YSB3093 | *MAT*α*fzc31*Δ:: *NAT-STM#273* | H99 | 51291 / KCCM |
| 572 | YSB3094 | *MAT*α*fzc31*Δ:: *NAT-STM#273* | H99 | 51291 / KCCM |
| 573 | YSB3300 | *MAT*α*gat201*Δ:: *NAT-STM#273* | H99 | 51291 / KCCM |
| 574 | YSB3301 | *MAT*α*gat201*Δ:: *NAT-STM#273* | H99 | 51291 / KCCM |

### Experimental Example 1. Analysis of STM-Based Murine Lung and Brain Infections Using Cryptococcus neoformans Kinase and Transcription Factor Mutant Libraries

To identify the kinases and transcription factors required for lung and brain infection, the lung-STM scores and the brain-STM scores for each mutant were compared using *Cryptococcus neoformans* kinase and transcription factor mutant libraries. For a method for preparing *Cryptococcus neoformans* kinase and transcription factor mutant libraries, Korean Patent Application Publication No. 10-2017-0054190 may be referred.

The high-throughput murine brain-infectivity test was performed using the transcription factor and kinase mutant libraries with the nourseothricin acetyltransferase (NAT) selection marker containing 46 unique signature tags (four and five groups of the transcription factor and kinase mutant libraries). The *ste50* mutants were used as virulent control strains.

Each group of libraries was incubated at 30°C in YPD medium for 16 hours, respectively, and washed three times with phosphate-buffered saline (PBS). The concentration of each mutant was adjusted to 10⁷ cells per ml⁻¹ and 50 µl of each sample was pooled into one tube.

For preparation of the input genomic DNA of each mutant library, 200 ml of the mutant pool was spread on YPD plate, incubated at 30°C for 3 days, and scraped. For preparation of the output genomic DNA samples, 50 µl of the mutant pool (5 × 10⁵ cells per mouse) was infected into seven-week-old female A/J mice (Japan SLC, Inc.) through intravenous (into the tail vein) or intracerebroventricular (ICV) injection. The intravenous injection was performed in warm (40°C) water for stimulating the expansion of the tail vein and the mice were immobilized with restraint devices. For ICV injection, mice were anaesthetised with 2% tribromoethanol (20 ml/kg, by intraperitoneal injection, Sigma Aldrich) and placed on a stereotaxic device (David Kopf Instruments). The control strain and mutant pool were injected into the ventricle (anteroposterior, -0.2 mm; lateral, -1.0 mm; ventral -2.0 mm) using a NanoFil needle (WPI) with Hamilton syringe and pump (WPI). The infected mice were sacrificed 7 days after infection and their brains were harvested. The brains were homogenised in 4 ml PBS, and then 200 µl of the samples was spread onto the YPD plates containing 100 mg per ml⁻¹ of chloramphenicol, incubated at 30°C for 2 days, and scraped. Total genomic DNA was extracted from scraped input and output samples by the cetrimonium bromide (CTAB) method.

Quantitative PCR was performed with the tag-specific primers, using CFX96^{™} Real-Time PCR detection system (Bio-Rad), and then the STM score was calculated. Relative changes in genomic DNA amounts were calculated by the 2^{-ΔΔCT} method to determine the STM score. The mean fold-changes in input versus output samples were calculated in Log score (Log₂ 2^{-(Ct,Target - Ct,Actin)output - (Ct,Target - Ct,Actin)input)}). Two independent mutants were identified for each kinase and transcription factor.

Using the same method, two independent mutants were identified for kinases and transcription factors using lungs harvested from intranasally infected mice (14 days after infection), and then brain-STM scores and lung-STM scores were compared.

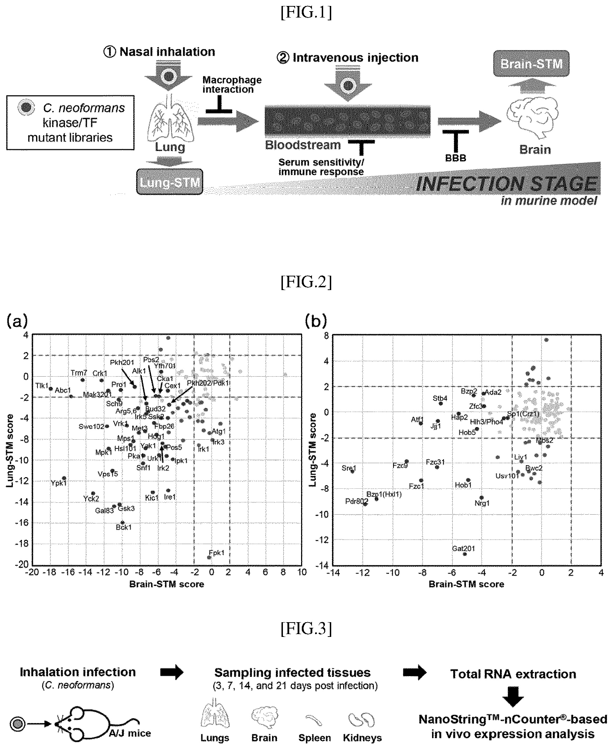

As a result, regarding kinases, the 34 kinases that were found to be required for both the lung and brain infections were defined as core-virulence kinases. The core-virulence kinases include: Pka1 in the cAMP signaling pathway, Ssk2 and Hog1 in the high osmolarity glycerol response (HOG) pathway, Bck1 and Mpk1 in the cell wall integrity MAPK pathway, Ire1 in the unfolded protein response (UPR) pathway, Vps15 in the vacuole-trafficking pathway, Snfl and Gal83 in the carbon utilisation pathway, Bud32 in the KEOPS/EKC complex, Ypk1, Gsk3, and Ipk1 in the TOR (Target of Rapamycin) pathway. Except for these known proteins, the core virulence kinases identified Irk2 and Irk5, whose functions *in vivo* are not evident. Irk2 and Irk5 belong to the families of APH phosphotransferases, diacylglycerol phosphatase-like kinase and AGC/YANK protein kinase, respectively. Deletion of *IRK5* significantly reduces melanin production, but dramatically enhances capsule production, indicating that defective melanin formation may be attributable to the role of Irk5 in virulence (FIG. 2).

In addition, in the case of transcription factors, a total of 9 transcription factors were found to be required for both lung and brain infections and here defined as core virulence transcription factors. It was identified that the core virulence transcription factors include Sre1 and Hob1 in the sterol biosynthesis pathway, and Gat201 and Nrg1 in the capsule biosynthetic pathway. In addition, Pdr802, Fzc1, Fzc9 and Fzc31, which all contain the fungal specific Zn₂Cys₆ DNA binding domain, were also identified as core-virulence transcription factors. Specifically, deletion of *FZC1* reduced the growth at 39°C (but not at 37°C) and mating capacity but increased capsule and melanin production. Deletion of *FZC9* reduced mating and resistance to hydrogen peroxide. Deletion of FZC31 reduced the growth under high temperature and oxidative stresses and mating capacity but increased melanin production.

From the above results, deletion of kinase genes resulted in more dramatic changes in both the lung and brain-STM scores than deletion of transcription factor genes did, because kinases generally function upstream of transcription factors in signaling pathways. These results indicate that redundant and distinct signaling components are involved in different cryptococcal infection stages.

### Experimental Example 2. In vivo Gene Expression Profiling of Cryptococcus Neoformans Infection-Related Virulence Genes, Kinases and Transcription Factors

To analyze the infection stage-dependent signaling pathway, NanoString nCounter-based *in vivo* transcription analysis was performed for 58 virulence-related factors, 180 transcription factors, and 183 kinases.

First, mice were intranasally infected with the *Cryptococcus Neoformans* H99 strain and infected lung, brain, kidney, and spleen tissues were harvested 3, 7, 14, and 21 days after infection. Total hosts and pathogen RNAs were isolated from each infected tissue and used for NanoString assays. Pathogen-specific mRNA transcripts were used to quantitate pathogen-specific mRNA transcripts using designed probes by normalization with average expression levels of eight housekeeping genes. *In vivo* expression levels of each target gene at different tissues and days of infection were compared to their *in vitro* expression levels under basal growth conditions [yeast extract-peptone-dextrose (YPD) medium at 30°C].

Specifically, six-week-old female A/J mice were infected with 5 × 10⁵ cells through nasal inhalation. After 3, 7, 14 or 21 days of infection, the lungs, brains, spleens and kidneys of 3 mice from each group were removed and lyophilized. Dried organs were homogenised and total RNA was extracted (from basal samples grown in YPD medium) by using total RNA extraction kit (easy-BLUE, Intron Biotechnology). Samples containing 10 ng of total RNA isolated from C. neoformans (from basal samples grown in YPD medium) or 10 µg of total RNA isolated from C. neoformans-infected mouse tissues were reacted with the designed probe code set designed according to the manufacturer's standard protocol.

A total of eight housekeeping genes were used for expression normalisation (mitochondrial protein, CNAG_00279; microtubule binding protein, CNAG_00816; aldose reductase, CNAG_02722; cofilin, CNAG_02991; actin, CNAG_00483; tubulin β chain, CNAG_01840; tubulin α-1A chain, CNAG_03787; histone H3, CNAG_04828). The normalised data was transformed to log₂ score to express the fold change and subject to clustering using one minus Pearson correlation with average linkage by Morpheus (https://software.broadinstitute.org/morpheus).

As a result, it was identified how 58 cell virulence-related genes are differentially regulated. Genes involved in metal ion sensing and uptake, such as *CIG1* and *CFO1,* were highly upregulated at different tissues at all infection stages, identifying that Cig1 and Cfo1 are essential for virulence of *Cryptococcus neoformans.* In addition, it was identified that the copper regulon genes such as *CnMT1*/*2* (metallothioneins) and *CTR4* (copper transporter) were also highly upregulated at all infected tissues from the early to the late infection stages. In addition, genes involved in the production of two major virulence factors, melanin (*LAC1*) and capsule (*CAP10, CAP59, CAP60, CAP64),* were differentially regulated during infection. Notably, it was identified that *in vivo* expression levels of *LAC1* increased during 3 to 14 days and decreased at 21 day, suggesting that the conditions for induction of *LAC1* are more favorable for haematogenous dissemination. CAP10, CAP59, CAP60 and CAP64 showed low overall expression levels, but only weakly increased expression in the lungs for 7 to 21 days.

In addition, the *in vivo* transcription profiling analysis of kinases and transcription factors revealed the expression patterns of 183 kinases and 180 transcription factors during the whole infection process. Reflecting that the lungs are the initial infection sites for *C. neoformans,* expression of a large number of kinases and TF genes were induced in the lungs, particularly after 14 days. It was identified that there were no genes specifically expressed in tissues other than the lung, and some genes exhibited high or low expression levels throughout the infection stage. In other words, it was identified that most genes involved in pathogenicity were generally highly expressed *in vivo.*

### Experimental Example 3. Identification of transcription factors and kinases required for adhesion and passage through the BBB

Since the brain is a lethal target tissue for *Cryptococcus neoformans* infection, the following experiment was performed focusing on transcription factor and kinase STM mutants that are specifically defective in brain infection. Unlike the lung-STM scores, the brain-STM scores were 12 and 10, respectively, for kinases and transcription factors showing a particularly low tendency, as shown in Table 3 below.

**[Table 3]**

| **Type** | | **Explanation** | **nasal inhalation** | | **IV** | **ICV** | |
|---|---|---|---|---|---|---|---|
| | ***S*. *cerevisiae* orthologue** | | **Lung STM** | **brain STM** | **brain STM** | **brain STM** | **BBB passage rate** |
| **Kinase** | | | | | | | |
| **Nutrient sensing/biosynthesis/glycolysis** | | | | | | | |
| Tlk1 | Tor1/Tor2 | Phosphatidylinositol 3-kinase has 57% similarity to ScTorl | 1.19 | 3.95 | **17.97^{∗}** | **12.82^{∗}** | 1.06 |
| Pro1 | Pro1 | Glutamate 5-kinase and ScPro1 are involved in proline biosynthesis | -1.30 | 2.71 | **10.12^{∗}** | 1.25 | ND |

| **Vacuolar trafficking/ER membrane assembly** | | | | | | | |
|---|---|---|---|---|---|---|---|
| Yfh701 | Yfh7 | P-loop kinase/ Phosphoribulokinase/Uridine kinase family, and ScYfh7 are involved in ER membrane assembly | 0.39 | **4.90^{∗}** | **5.64^{∗}** | 1.32 | **0.50^{∗}** |

| **Fungal cell wall integrity** | | | | | | | |
|---|---|---|---|---|---|---|---|
| Pkh201 | - | Serine/threonine protein kinase; With 28% similarity to ScPkh2. ScPkh2 is involved in sphingolipid-mediated signaling pathways that control endocytosis by regulating lysosome assembly and tissues | 1.03 | -5.35 | **-.61^{∗}** | **-6.16^{∗}** | **0.57^{∗}** |

| **Cell growth and proliferation/developmental process** | | | | | | | |
|---|---|---|---|---|---|---|---|
| Ckal | Cka1/Cka2 | Alpha subunit of casein kinase 2 (CK2); serine/threonine protein kinase; Involved in cell growth and proliferation | 1.93 | **5.08^{∗}** | **5.90^{∗}** | 0.70 | ND |
| Alk1 | Alk1 | Haspin protein kinase and ScAlk1 are phosphorvlated in response to DNA damage | 2.61 | **4.36^{∗}** | **7.30^{∗}** | **-5.74^{∗}** | **0.39^{∗}** |
| Crk1 | Ime2 | CMGC/RCK protein kinase involved in the binding process | 0.39 | -2.68 | **12.24^{∗}** | **11.47^{∗}** | 1.12 |

| **Stress response/HOG MAPK cascade** | | | | | | | |
|---|---|---|---|---|---|---|---|
| Pbs2 | Pbs2 | STE/STE7 protein kinase involved in the HOG pathway | 1.90 | **2.20^{∗}** | **6.25^{∗}** | -1.08 | **0.47^{∗}** |

| **tRNA modification** | | | | | | | |
|---|---|---|---|---|---|---|---|
| Trm7 | Trm7 | CAMK protein kinase and ScTrm7 are Trna methyl transferases | 0.36 | -4.17 | **14.40^{∗}** | **11.69^{∗}** | 0.96 |
| Cex1 | Cex1 | SCYL protein kinase and ScCex1 are components of the tRNA export pathway | -1.40 | -3.56 | **4.93^{∗}** | 0.48 | **0.12^{∗}** |

| **Double-stranded RNA replication** | | | | | | | |
|---|---|---|---|---|---|---|---|
| Mak3201 | Mak32 | PfkB family carbohydrate kinases | -1.37 | -3.06 | **11.53^{∗}** | **10.24^{∗}** | 1.15 |

| **Unknown functions** | | | | | | | |
|---|---|---|---|---|---|---|---|
| Abel | - | Atypical/ABC1 protein kinase | -1.95 | -5.59 | **15.68^{∗}** | -1.94 | 0.85 |

| **Transcription factor (TF)** | | | | | | | |
|---|---|---|---|---|---|---|---|
| **Nutrient sensing/metabolism pathway** | | | | | | | |
| Hap2 | Hap2 | ScHap2 is a subunit of the CCAAT-binding complex that inhibits heme activation and glucose | -0.15 | 0.29 | **-5.54^{∗}** | **14.82^{∗}** | **0.24^{∗}** |
| Sp1(Crz1) | Crz1 | Downstream of the Calcineurin pathway; Involved in toxicity, cell wall integrity/membrane stability in C. *neoformans* | -0.49 | -0.75 | **-2.26^{∗}** | 1.33 | 1.25 |

| **Stress response/fungal infectivity** | | | | | | | |
|---|---|---|---|---|---|---|---|
| Ada2 | Ada2 | SAGA complex that regulates histone acetylation; related to toxicity of C. *neoformans* | 1.45 | **-3.84^{∗}** | **-3.86^{∗}** | **17.39^{∗}** | **0.29^{∗}** |
| Atf1 | Sko1 | bZIP transcription factor; Involved in osmotic and oxidative stress responses in C. *neoformans* | -0.90 | **2.88^{∗}** | **-8.10^{∗}** | 1.22 | 0.81 |

| **Ribosome biogenesis** | | | | | | | |
|---|---|---|---|---|---|---|---|
| Jjj1 | Jii1 | DNAJ-like cochaperone; U1 snRNA-type Zn finger function in 60S ribosomal subunit biogenesis; related to toxicity of C. *neoformans* | -0.73 | -1.25 | **-6.94^{∗}** | -1.24 | **0.41^{∗}** |

| **Phosphate sensing and acquisition** | | | | | | | |
|---|---|---|---|---|---|---|---|
| Pho4/Hlh3 | - | TF, a helix-loop-helix DNA-binding domain, and Pho4 are involved in phosphate uptake and stress tolerance at alkaline pH and is essential for C. *neoformans* propagation in the host brain | -0.51 | -0.97 | **-2.53^{∗}** | 0.64 | **0.53^{∗}** |

| **Unknown functions** | | | | | | | |
|---|---|---|---|---|---|---|---|
| Bzp2 | - | DNA-binding (glucocorticoid receptor-like; base region leucine zipper; GATA zinc finger) | 1.29 | 1.99 | **-4.56^{∗}** | **14.82^{∗}** | ND |
| Zfc3 | - | TF, C₂H₂-type zinc finger | 0.44 | **-8.43^{∗}** | **-3.83^{∗}** | **14.41^{∗}** | 1.27 |
| Stb4 | - | TF, fungal(2)-Cys(6) binuclear cluster domain | 0.67 | 0.49 | **-6.77^{∗}** | -0.05 | 0.95 |
| Hob5 | - | TF, homeo domain-like; helix-turn-helix domain | -1.36 | ND | **-4.32^{∗}** | 1.45 | 0.83 |

| | | | | | | | |
|---|---|---|---|---|---|---|---|
| ^{∗} ND: Among the undetermined causes of growth defects in deletion mutants at human temperature, it was identified whether brain-STM score changes occurred during haematogenous dissemination, and only the *pho4Δ* mutant exhibited serum-specific growth defects. It was hypothesized that some of the remaining mutants may be involved in passage through the brain-blood barrier covering the brain. In order identify whether these 12 kinases and 10 transcription factors are essential for adhesion and passage through the brain-blood barrier, experiments were performed using the BBB system *in vitro.* | | | | | | | |

In addition, there are 34 kinases and 9 transcription factors that tend to be low in both brain-STM scores and lung-STM scores, respectively, as shown in Table 4 below.

**[Table 4]**

| **Type** | ***S. cerevisiae* orthologue** | **Explanation** | **nasal inhalation** | | **IV** | **ICV** | **BBB passage rate** |
|---|---|---|---|---|---|---|---|
| | | | **Lung STM** | **brain STM** | **brain STM** | **Lung STM** | |
| **kinase** | | | | | | | |
| **Nutrient sensing/biosynthesis/glycolysis** | | | | | | | |
| Yck2 | Yck1/2 | Casein Kinase I (CK1) homologue; ScYckl functions in intracellular transport and glucose sensing | **11.18^{∗}** | **13.19^{∗}** | **13.23^{∗}** | **19.12^{∗}** | ND |
| Arg5,6 | Arg5,6 | Acetylglutamate kinase; ScArg5,6 is the third stage in the biosynthesis of organan | **-3.05^{∗}** | **-8.32^{∗}** | **-8.24^{∗}** | **18.25^{∗}** | ND |
| Met3 | Met3 | ScMet3 catalyzes the first stage of intracellular sulfate activation involved in methionine metabolism | **-5.27^{∗}** | **-9.71^{∗}** | **-7.47^{∗}** | **17.55^{∗}** | **0.13^{∗}** |
| Gal83 | Gal83 | Beta-subunit of Snfl kinase complex; ScGal83 is involved in galactose metabolism | **12.37^{∗}** | **13.95^{∗}** | **10.85^{∗}** | **17.14^{∗}** | **0.40^{∗}** |
| Urk1 | Urk1 | Uridine/cytidine kinase; ScUrk1 is involved in the pyrimidine ribonucleotide salvage pathway | **-7.51^{∗}** | **11.19^{∗}** | **-5.91^{∗}** | **15.69^{∗}** | **0.41^{∗}** |
| Yak1 | Yak1 | ScYak1 is a serine/threonine protein kinase that is a component of the glucose-sensing system | **-6.86^{∗}** | **-9.83^{∗}** | **-7.39^{∗}** | **14.62^{∗}** | 0.87 |
| Snfl | Snfl | ScSnf1 is an AMP-activated serine/threonine protein kinase involved in alternative carbon source utilization | **-8.32^{∗}** | **16.80^{∗}** | **-7.71^{∗}** | **13.08^{∗}** | **0.27^{∗}** |
| Fbp26 | Fbp26 | ScFbp26 is fructose-2,6-bisphosphatase required for glucose metabolism | **-4.46^{∗}** | **-8.63^{∗}** | **-6.43^{∗}** | **12.70^{∗}** | ND |

| **Cell cycle/Spindle checkpoint** | | | | | | | |
|---|---|---|---|---|---|---|---|
| Mps1 | Mps1 | ScMps1 is a dual specificity kinase required for spindle checkpoint function | **-6.17^{∗}** | **-6.20^{∗}** | **-8.76^{∗}** | **15.65^{∗}** | ND |
| Hsl101 | Hsll | ScHsl1 is a septin-binding kinase located in the bud neck septin loop and regulating the morphogenesis checkpoint | **-6.54^{∗}** | **12.24^{∗}** | **-9.07^{∗}** | **15.57^{∗}** | **0.23^{∗}** |
| Mecl | Mecl | ScMec1 is a member of the genomic integrity checkpoint protein and PI kinase superfamily required for cell cycle arrest | **-7.59^{∗}** | **-9.87^{∗}** | **-5.04^{∗}** | **11.01^{∗}** | ND |
| Swe102 | - | The ScSwe1 protein regulates the G2/M transition; Positive regulator of sphingolipid biosynthesis through Orm2p | **-4.80^{∗}** | **-8.93^{∗}** | **11.68^{∗}** | **-6.97^{∗}** | ND |
| Cdc7 | Cdc7 | ScCdc7 is a catalytic subunit of the serine/threonine kinase and DDK (Dbf4-dependent kinase) complex for kinetochores during meiosis I. Null mutants are inevitable | **-5.43^{∗}** | **-5.98^{∗}** | **-8.15^{∗}** | **-** | ND |
| Gsk3 | Rim11/Mrk1 | Gsk3 is a CMGC/GSK protein kinase involved in normal growth and virulence in *C. neoformans* | **12.22^{∗}** | **10.23^{∗}** | **10.30^{∗}** | **-** | ND |

| **Fungal cell wall/membrane integrity** | | | | | | | |
|---|---|---|---|---|---|---|---|
| Kic1 | Kiel | Kiel is a component of the RAM pathway involved in cell polarity, morphogenesis and cell integrity | **11.09^{∗}** | **15.83^{∗}** | **-6.56^{∗}** | **15.34^{∗}** | ND |
| Mpk1 | Mpk1 | Mpk1 is a MAPK in the PKC1-mediated signaling pathway involved in cell wall biosynthesis and regulation | **-6.89^{∗}** | **10.35^{∗}** | **11.46^{∗}** | **15.75^{∗}** | ND |
| Bck1 | Bck1 | Bck1 is a MAPKKK in the PKC1-mediated signaling pathway involved in cell integrity | **13.94^{∗}** | **15.12^{∗}** | **-9.94^{∗}** | **13.64^{∗}** | ND |
| Ypkl | Ypk1/Ypk2 | ScYpkl is a serine/threonine protein kinase that down-regulates ScFpkl, a flippase activator; Involved in TORC-dependent phosphorylation. The ypk1Δ mutant exhibits strongly defective melanogenesis and non-toxicity in a murine Cryptococcus model | **-9.74^{∗}** | **-8.58^{∗}** | **16.48^{∗}** | **-** | ND |

| **Phospholipid metabolism** | | | | | | | |
|---|---|---|---|---|---|---|---|
| Pkh202/Pdk1 | Pkh1/Pkh2 | Pdkl is a serine/threonine kinase; Involved in fluconazole resistance through Pkcl and Ypkl by regulating sphingolipid homeostasis | **-2.74^{∗}** | -**8.51^{∗}** | **-4.78^{∗}** | -3.71 | ND |
| Pka1 | Tpk2/Tpk3 | Pka1 is involved in inositol and phospholipid metabolism and continuous capsule production | **-7.55^{∗}** | **12.83^{∗}** | **-7.65^{∗}** | -5.20 | 1.15 |

| **Stress response/HOG MAPK cascade** | | | | | | | |
|---|---|---|---|---|---|---|---|
| Hog1 | Hog1 | Involved in HOG hyperosmolar glycerol pathway MAPK response to osmotic stress | **-5.60^{∗}** | **-8.67^{∗}** | **-6.14^{∗}** | **-7.31^{∗}** | 0.90 |
| Dak101 | Dak1/Dak2 | ScDak1 is a dihydroxyacetone kinase; Necessary for detoxification of dihydroxyacetone (DHA); Involved in stress adaptation | **-2.67^{∗}** | -1.63 | **-3.14^{∗}** | **-** | 0.97 |
| Ssk2 | Ssk2 | HOG pathway MAPKKK(Ssk2 MAPKKK-Pbs2 MAPKK-Hog1 MAPK) | **-4.01^{∗}** | **-8.24^{∗}** | **-5.66^{∗}** | **4.38^{∗}** | 0.97 |

| **Redox reactions** | | | | | | | |
|---|---|---|---|---|---|---|---|
| Utrl | Utrl | ATP-NADH kinase; Involved in phosphorvlation of NAD and NADH | **-3.98^{∗}** | **-4.31^{∗}** | **-4.92^{∗}** | **13.47^{∗}** | ND |
| Pos5 | Pos5 | Involved in NADH phosphorylation, and mitochondrial NADH kinase | **-6.71^{∗}** | **11.74^{∗}** | **-5.17^{∗}** | **11.11^{∗}** | ND |

| **Ribosome biogenesis/ribosomal RNA processing** | | | | | | | |
|---|---|---|---|---|---|---|---|
| Sch9 | Sch9 | ScSch9 is involved in the regulation of sphingolipid biosynthesis. Sch9 regulates heat sensitivitv in *C. neoformans* | **-2.25^{∗}** | **-8.78^{∗}** | **10.35^{∗}** | -1.72 | **0.34^{∗}** |
| Vrk1 | - | 1-phosphatidylinositol-3-phosphate 5-kinase; Translation and ribosomal RNA processing | **-4.72^{∗}** | **12.93^{∗}** | **-9.50^{∗}** | **12.46^{∗}** | **0.38^{∗}** |

| **Unfolded protein response** | | | | | | | |
|---|---|---|---|---|---|---|---|
| Ire1 | Ire1 | Ire1 is a serine/threonine protein kinase that mediates unfolded protein responses and is important for virulence in *C. neoformans* | **10.92^{∗}** | **-4.19^{∗}** | **-4.85^{∗}** | **-4.77^{∗}** | ND |
| Kin1 | Kin1 | Polarized exocytosis; ScKin1 is involved in Ire1p-mediated protein responses | **-2.38^{∗}** | **-9.27^{∗}** | **-2.75^{∗}** | **16.95^{∗}** | 1.02 |

| **Inositol polyphosphate biosynthetic process** | | | | | | | |
|---|---|---|---|---|---|---|---|
| Ipk1 | - | Inositol 1, 3, 4, 5, 6-pentakisphosphate 2-kinase; a nuclear protein required for the synthesis of 1, 2, 3, 4, 5, 6-hexakisphosphate (phytate) | **-7.90^{∗}** | **10.21^{∗}** | **-4.34^{∗}** | **-9.93^{∗}** | ND |

| **Protein sorting** | | | | | | | |
|---|---|---|---|---|---|---|---|
| Vps15 | Vps15 | ScVps15 is a serine/threonine protein kinase involved in vacuum protein classification | **-9.00^{∗}** | **10.55^{∗}** | **11.04^{∗}** | - | ND |

| **tRNA modification** | | | | | | | |
|---|---|---|---|---|---|---|---|
| Bud32 | Bud32 | ScBud32 is a serine/threonine protein kinase involved in vacuum protein classification | **-3.38^{∗}** | **15.65^{∗}** | **-7.08^{∗}** | **14.06^{∗}** | ND |

| **Unknown functions** | | | | | | | |
|---|---|---|---|---|---|---|---|
| Irk2 | - | Infection-related kinase 2 | **-6.40^{∗}** | **-3.05^{∗}** | **-5.54^{∗}** | **13.83^{∗}** | **0.48^{∗}** |
| Irk5 | - | Infection-related kinase 5 | **-3.61^{∗}** | **-7.76^{∗}** | **-7.32^{∗}** | 0.68 | **0.35^{∗}** |

| **Transcription factor (TF)** | | | | | | | |
|---|---|---|---|---|---|---|---|
| **Nutrient sensing** | | | | | | | |
| Nrg1 | Nrg1 | ScNrg1 is involved in glucose inhibition; Involved in filamentous growth and alkaline pH response | **-6.73^{∗}** | **-8.56^{∗}** | **-4.01^{∗}** | **-9.26^{∗}** | ND |

| **Fatty acid and spingolipid biosynthesis** | | | | | | | |
|---|---|---|---|---|---|---|---|
| Sre1 | - | Sterol regulatory element-binding protein; Involved in melanin and toxicity in *C. neoformans* | **-4.64^{∗}** | **10.23^{∗}** | **12.70^{∗}** | **17.10^{∗}** | **0.07^{∗}** |
| Hob1 | - | Homeobox domain included | **-5.34^{∗}** | **-9.36^{∗}** | **-4.91^{∗}** | **-7.64^{∗}** | **0.38^{∗}** |

| **Virulence factor regulation** | | | | | | | |
|---|---|---|---|---|---|---|---|
| Gat201 | - | GATA-family zinc finger DNA-binding transcriptional regulators; Involved in capsule and melanogenesis, capsule-independent antiphagocytic function and toxicity in *C. neoformans* | **11.13^{∗}** | **-5.67^{∗}** | **-5.13^{∗}** | **12.52^{∗}** | 1.26 |

| **Unfolded protein response** | | | | | | | |
|---|---|---|---|---|---|---|---|
| Bzpl(Hxll) | - | Transcription factor downstream of Ire1 in the unfolded protein response; Essential for toxicity in *C. neoformans* | **-6.82^{∗}** | **-7.44^{∗}** | **11.09^{∗}** | -2.80 | ND |

| **Unknown functions** | | | | | | | |
|---|---|---|---|---|---|---|---|
| Fzc1 | - | Fungal Zn(2)-Cys(6) is a transcription factor with a binuclear cluster domain | **-5.37^{∗}** | - | **-8.09^{∗}** | -0.51 | **0.28^{∗}** |
| Fzc9 | - | Fungal Zn(2)-Cys(6) is a transcription factor with a binuclear cluster domain | **-3.86^{∗}** | **-6.42^{∗}** | **-9.06^{∗}** | -1.09 | **0.14^{∗}** |
| Fzc31 | - | Fungal Zn(2)-Cys(6) is a transcription factor with a binuclear cluster domain | **-4.33^{∗}** | **11.23^{∗}** | **-7.02^{∗}** | -4.53 | 1.16 |
| Pdr802 | - | Fungal Zn(2)-Cys(6) is a transcription factor with a binuclear cluster domain | **-7.21^{∗}** | **19.38^{∗}** | **11.84^{∗}** | **19.14^{∗}** | **0.23^{∗}** |

| | | | | | | | |
|---|---|---|---|---|---|---|---|
| ^{∗} ND: The undetermined causes of growth defects in deletion mutants at human temperature | | | | | | | |

### 3-1. Validation of the in vitro BBB system

First, to verify the *in vitro* BBB system, in order to compare the brain-blood barrier passage ability of *Cryptococcus neoformans (Cryptococcus neoformans* H99 strain), avirulent *Saccharomyces cerevisiae (Saccharomyces cerevisiae* S288C) and the *mpr1Δ* mutant independently constructed in the present invention, after incubating the strain for 24 hours, it was identified whether it could pass through the brain-blood barrier.

As a result, about 10% of the wild-type *Cryptococcus neoformans* were able to pass through the brain-blood barrier. In contrast, *S. cerevisiae* (S288C) did not pass through the brain-blood barrier at all and the *mpr1Δ* mutant barely passed through the brain-blood barrier (FIG. 5A). In addition, as a result of trans-endothelial electrical resistance (TEER) measurement, any significant change was not found, further verifying that the tight junction joining neighboring HBMECs was not affected during the brain-blood barrier passage of *Cryptococcus neoformans* cells.

These results suggest that the *in vitro* BBB system works as expected.

### 3-2. Identification of Transcription Factors and Kinases Required for Adhesion and Passage through Brain-Blood Barrier using in vitro BBB System

After validating and establishing the BBB system as above, the following experiments were performed to identify key toxic kinases and transcription factors in the signaling pathway of 10 transcription factors related to brain infection selected in passage through the brain-blood barrier, 12 kinase mutants and 9 transcription factors identified as important for both brain and lung infections, and *CEX1, ALK1, PBS2, YFH701, PKH201, ABC1, TRM7, TLK1, MAK3201, CRK1, HAP2, ADA2, JJJ1, PHO4, ATF1, HOB5, STB4, CRZ1, ZFC3, MET3, HSL101, SNF1, SCH9, IRK5, VRK1, GAL83, URK1, IRK2, YAK1, HOG1, SSK2, DAK101, KIN1, PKA1, SRE1, FZC9, PDR802, FZC1, HOB1, FZC31* and *GAT201* gene mutated strains except for defective signaling pathways in growth at 37°C among 34 kinases, through a transwell-based *in vitro* BBB system. Human brain microvascular endothelial cells (HBMECs) on transwells separating the upper compartment of the blood region and the lower compartment of the brain region were used for BBB screening.

First, for endothelial cell line culture, hCMECM/D3 cells were seeded on collagen coated 8 µm porous membranes (BD Falcon) or 12-well plates (BD Falcon) at a density of 5 × 10⁴ cells/mL and maintained in EGM^{™}-2 (Longa). The day after seeding, medium was replaced with 2.5% human serum, and cultured for 4 days. 24 hours before culturing, the medium was replaced with 0.5× diluted medium and the cells were maintained at 37°C and 5% CO₂ (transendothelial electrical resistance (TEER) around 200 Ω/cm²).

For *in vitro* BBB screening, 5 × 10⁵ cells of *Cryptococcus neoformans* WT, deletion mutants, or *S. cerevisiae* strains were added to 500 µl of PBS and inoculated onto the top of the porous membrane. After 24 hours of incubation at 37°C in CO₂ incubator, the number of cells passing through the membrane was measured by counting CFU. For adhesion assays, the inoculated 24-h culture dishes were washed three times with PBS and reacted with sterile distilled water for 30 minutes in a 37°C incubator to burst and collect hCMEC/D3 cells. The degree of brain-blood barrier migration or endothelial cell adhesion was calculated at a ratio of CFU to the output WT. TEER was measured by using an EVOM² device (World Precision Instruments) before and after inoculation of yeast cells.

As a result, it was identified that a total of 5 kinases (Cex1, Alk1, Pbs2, Yfh701, and Pkh201) and 4 transcription factors (Ada2, Hap2, Pho4/Hlh3, and Jjj1) were required for the brain-blood barrier passage (FIG. 5B). Moreover, the capability of the brain-blood barrier passage defective 5 kinases and 4 TF mutants to adhere to the monolayer of HBMECs was identified. It was identified that most mutants except *jjj1Δ, yfh701*Δ, *ada2Δ,* and *pho4A* mutants showed reduced adhesion to HBMECs (FIG. 6A).

In addition, Sre1 and Hob1 in the sterol biosynthesis pathway, Sch9 in the TOR pathway, Met3 in the sulphur assimilation pathway, Snfl and Gal83 in the carbon utilisation pathway, and Vrk1 in ribosome biogenesis pathway were identified to promote brain-blood barrier passage of *Cryptococcus neoformans* (FIG. 5C), and Hsl101, Irk2, Irk5, Urk1, Fzc1, Fzc9 and Pdr802 were also identified to be critical for brain-blood barrier passage. Furthermore, the *met3*Δ*, snf1*Δ*, vrk1*Δ*, gal831*Δ*, hsl101*Δ*, irk2*Δ*, sre1*Δ*, fzc9*Δ*, hob1*Δ*, pdr802*Δ*,* and *fzc1*Δ mutants showed reduced adhesion to the monolayer of HBMECs (FIG. 6B).

These results suggest that the adhesion of host cells is critical in the efficient brain-blood barrier passage of *Cryptococcus neoformans,* and that *Cryptococcus neoformans* employs complex signaling networks involved in a variety of biological processes to pass through the brain-blood barrier.

### 3.3. Identification of in vivo Transcription Profiling Analysis of Transcription Factors and Kinases Required for Brain-Blood Barrier Adhesion and Passage

The determination of whether the transcription factors and kinases identified in Experimental Example 3-2 are specifically regulated only in the brain was identified using the NanoString nCounter-based *in vivo* transcription analysis of Experimental Example 2 above.

As a result, it was identified that most of these genes were upregulated in the lungs at the later stage of infection. Particularly *in vivo* expression of *PDR802, SRE1, VRK1, PKH201,* and *YFH701* was strongly induced in all infected tissues tested during almost all infection stages. In contrast, regardless of the critical roles of Cex1 and Met3 in BBB passage and adhesion to HBMECs, their *in vivo* expression levels were not strongly induced at all infection stages and in all tissues.

### Experimental Example 4. Identification of Kinases and Transcription Factors Required for Survival of Cryptococcus Neoformans inside Brain

To elucidate why some kinases and transcription factors were normal in haematogenous dissemination and passing through the brain-blood barrier, but still showed changes in brain-STM score, an experiment was performed to monitor the capability of the transcription factors and kinase mutants of low brain-STM scores for proliferation inside the brain.

### 4-1. Identification of Transcription Factors and Kinase Mutants of Low Brain-STM Scores

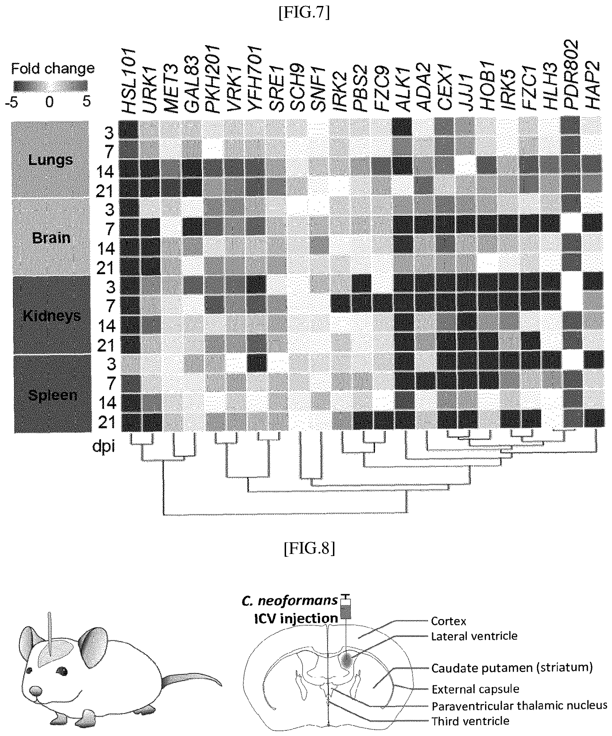

First, a new intracerebroventricular (ICV) injection method was established to infect the mouse brain with *Cryptococcus neoformans* by bypassing the brain-blood barrier (FIG. 8). Once a group of mice was infected with the high/low brain-STM transcription factors and kinase mutants by ICV injection, mutants were harvested from the infected brain after 6 dpi and assessed the STM score by qPCR (hereinafter, abbreviated as ICV-STM score).

As a result, it was found that strains with mutated 6 kinases (*TLK1, TRM7, CRK1, MAK3201, PKH201,* or *ALK1*) and 4 transcription factors *(ADA2, BZP2, ZFC3,* or *HAP2* gene) displayed significantly reduced ICV-STM score (FIG. 9).

Therefrom, it was identified that 2 kinases (Pkh201 and Alk1) and 2 transcription factors (Ada2 and Hap2) were required for both brain-blood barrier passage and survival inside the brain, whereas the kinases and transcription factors that were required for brain-blood barrier passage (Cex1, Pbs2, Yfh701, Pho4, and Jjj1) were dispensable for proliferation inside the brain.

In addition, 4 kinases (Tlk1, Trm7, Crk1, and Mak3201) and a single transcription factor (Zfc3) were uniquely involved in proliferation inside the brain, but not in brain-blood barrier passage.

### 4-2. Identification of Additional Kinases and Transcription Factors Required for Proliferation of Cryptococcus Neoformans inside Brain

To identify other kinases and transcription factors functionally related to those required for the proliferation of *Cryptococcus neoformans* inside the brain, ICV-STM scores for key virulence kinases and transcription factor mutants were identified.

As a result, most kinase and transcription factor mutants that were defective in growth at 37°C (*yck2*Δ*, arg-5*/*6*Δ*, mpk1*Δ*, mps1*Δ*, kic1*Δ*, bud32*Δ*, bck1*Δ*, utr1*Δ, *fbp26*Δ*, pos5*Δ*, mec1*Δ*, ipk1*Δ*, swe102*Δ*,* and *nrg1*Δ) also showed reduced ICV-STM score. Among the core kinases and transcription factors that were required for brain-blood barrier passage, Irk2, Vrk1, Pdr802, Srel, and Hob1 were also identified to be required for proliferation inside the brain (FIG. 9).
^{∗} *hog1*Δ mutant showed low ICV-STM score, indicating that Hog1 is not required for brain-blood barrier passage, but required for proliferation inside the brain. In contrast, Sch9, Irk5, Fzc1, and Fzc9 were dispensable for proliferation inside the brain. In addition, Snfl, Gal83, Kin1, Urk1, Hsl101, Yak1, Fbp26, Pos5, and Swe102 were identified to be involved in the proliferation of *Cryptococcus neoformans* inside the brain.

The above results suggest that *Cryptococcus neoformans* employs redundant and distinct sets of signaling pathways to pass through the brain-blood barrier and proliferate inside the brain parenchyma.

The above description is merely illustrative of the present invention, and one of ordinary skill in the art to which the present invention pertains should understand that the present invention may be easily modified in other specific forms without changing the technical spirit or essential features of the present invention. Therefore, the embodiments described above are illustrative in all aspects and should not be understood as limiting. For example, each element described as having an integrated form may be embodied in a distributed manner, and likewise, elements which are described as being distributed may be embodied in an integrated form.

The scope of the present invention is defined by the claims below, and all modifications or modified forms derived from the meaning and scope of the claims and concepts equivalent thereto should be interpreted as belonging to the scope of the present invention.

## Claims

1. A method for screening a fungal brain-blood barrier (BBB) passage inhibitor, wherein the method includes:
(a) contacting a sample to be analyzed with *Cryptococcus neoformans* cells containing any one or more proteins selected from the group consisting of Cex1, Alk1, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1;
(b) measuring an amount or activity of the protein; and
(c) discriminating that the sample is a fungal BBB passage inhibitor when it is measured that the amount or activity of the protein is down-regulated in stage (b).

2. The method of claim 1, wherein stages (a) and (b) are performed at 30°C to 40°C.

3. An antifungal composition including an inhibitor screened according to the method of claim 1.

4. The antifungal composition of claim 3, wherein the inhibitor is any one or more of an antibody, a dominant-negative mutation, and a ribozyme against a protein involved in passage through the brain-blood barrier (BBB).

5. The antifungal composition of claim 3, wherein the inhibitor is an antisense oligonucleotide, siRNA, shRNA, miRNA, or a vector containing the same for a gene encoding a protein involved in passage through the brain-blood barrier (BBB).

6. A pharmaceutical composition for preventing, treating, or preventing and treating meningoencephalitis or cryptococcosis, wherein the composition includes an antifungal composition according to any one of claims 3 to 5 as a pharmacologically active ingredient.

7. The pharmaceutical composition of claim 6, further including an azole-based or non-azole-based antifungal agent.

8. The pharmaceutical composition of claim 7, wherein the azole-based antifungal agent is one or more of fluconazole, itraconazole, voriconazole, and ketoconazole.

9. The pharmaceutical composition of claim 7, wherein the non-azole-based antifungal agent is amphotericin B or fludioxonil.

10. The antifungal composition of any one of claims 3 to 5, wherein the composition is a cosmetic composition.

11. A method for screening a bacterial or fungal brain-blood barrier passage inhibitor, wherein the method includes:
treating a sample to be analyzed with any one or more proteins involved in passage through a brain-blood barrier selected from the group consisting of Cex1, Alk1, Pbs2, Yfh701, Pkh201, Met3, Hsl101, Snfl, Sch9, Irk5, Vrk1, Gal83, Urk1, Irk2, Ada2, Hap2, Pho4/Hlh3, Srel, Fzc1, Pdr802, Fzc9, Hob1, and Jjj1; and
analyzing an amount or activity of any one or more of the proteins.

12. An antifungal composition including an inhibitor screened according to the method of claim 11.

13. The antifungal composition of claim 11, wherein the inhibitor is any one or more of an antibody, a dominant-negative mutation, and a ribozyme against a protein involved in passage through the brain-blood barrier (BBB).

14. The antifungal composition of claim 11, wherein the inhibitor is an antisense oligonucleotide, siRNA, shRNA, miRNA, or a vector containing the same for a gene encoding a protein involved in passage through the brain-blood barrier (BBB).

15. A pharmaceutical composition for preventing, treating, or preventing and treating meningoencephalitis or cryptococcosis, wherein the composition includes an antifungal composition according to any one of claims 12 to 14 as a pharmacologically active ingredient.

16. The antifungal composition of any one of claims 12 to 14, wherein the composition is a cosmetic composition.
